# FASCICULE DE BREVET EUROPEEN

(11) **EP 3 562 472 B1**
(45) Date de publication et mention de la délivrance du brevet: **19.05.2021**
(21) Numéro de dépôt: 17832534.6
(22) Date de dépôt: 22.12.2017
(51) Int. Cl.: A61K 8/97, B01D 11/00, A61K 36/00, A23L 33/105

(54) **UTILISATION DE L'EAU DE COCO COMME SOLVANT D'EXTRACTION**
VERWENDUNG VON KOKOSNUSSWASSER ALS EXTRAKTIONSLÖSUNGSMITTEL
USE OF COCONUT WATER AS EXTRACTION SOLVENT

(30) Priorité: 29.12.2016 FR 1663506
(43) Date de publication de la demande: 06.11.2019
(73) Titulaire: BASF Beauty Care Solutions France SAS, 69007 Lyon (FR); Universite D'Avignon et Des Pays Du Vaucluse, 84029 Avignon (FR)
(72) Inventeur: MAKERRI, Caroline, 84000 Avignon (FR); MOSER, Philippe, 54130 Dommartemont (FR); CHEMAT, Farid, 84310 Morrières les Avignon (FR); PERINO, Sandrine, 84140 Montfavet (FR)
(74) Mandataire: Cabinet Becker et Associés
(86) Numéro de dépôt international: PCT/FR2017/053809
(87) Numéro de publication internationale: WO 2018/122514

(56) Documents cités:
- EP-A1- 1 913 821
- JP-A- 2006 052 191
- US-A1- 2008 317 891
- US-A1- 2016 122 706
- US-B2- 8 632 829

## Description

### DOMAINE DE L'INVENTION

La présente invention est relative à un nouveau solvant d'extraction naturel d'origine végétale, et son utilisation pour l'extraction de matière premières naturelles, notamment d'origine végétale.

### ARRIERE-PLAN TECHNOLOGIQUE DE L'INVENTION

Dans les industries chimiques, alimentaires, pharmaceutiques et cosmétiques, les solvants organiques sont largement utilisés pour l'extraction à partir de matières premières végétales de principes actifs ou de molécules (extraits totaux, arômes, parfums, colorants, métabolites primaires ou secondaires). Selon la nature des composés ciblés, des solvants tels que les alcools, l'hexane et l'acétate d'éthyle, sont généralement utilisés dans ces procédés extractifs. Cependant, ces solvants peuvent être toxiques, inflammables, explosifs ou peu biodégradables.

Des réglementations toujours plus nombreuses et contraignantes concernant les normes d'émissions de polluants atmosphériques (Composés Organiques Volatils ou autres), la santé humaine (substances classées Cancérigènes, Mutagènes, Reprotoxiques) ou l'environnement imposent à terme leur substitution par de nouveaux solvants alternatifs biodégradables et peu toxiques, et pouvant présenter une capacité de solubilisation large en termes de sélectivité et de polarité.

Le remplacement des solvants issus de la pétrochimie est donc un défi majeur des dernières décennies. Pourtant, à ce jour, peu de candidats permettent d'atteindre les faibles coûts de production et les hauts rendements obtenus avec les solvants conventionnels. À cela s'ajoutent les contraintes réglementaires imposées pour l'enregistrement de nouvelles substances chimiques.

Pour pallier ce problème, l'utilisation de substances naturelles reste la meilleure alternative et la recherche bio-inspirée apparaît donc essentielle.

Plusieurs types de solvants parmi lesquels les liquides ioniques (LIs) et les solvants eutectiques profonds (SEPs ou DES) ont été suggérés comme solutions alternatives pour remplacer les solvants organiques volatils.

Le terme de liquides ioniques (LIs) apparaît pour la première fois dans les années 50. Depuis, les recherches sur ce type de solvants ont augmenté de façon exponentielle. En effet, cet engouement est dû aux propriétés remarquables des LIs qui les rendent beaucoup plus attrayants que les solvants organiques conventionnels. Outre leur haute stabilité thermique, ils possèdent une pression de vapeur faible, sont ininflammables et électrochimiquement stables. La définition commune acceptée d'un LI est qu'il s'agit d'un fluide ionique issu d'un sel fondu dont la température est en dessous ou proche de 100°C (Moutiers G, Isabelle Billard. Les liquides ioniques : des solvants pour l'industrie. Techniques de l'Ingénieur. Janvier 2005, AF 6 712,1-18).

Historiquement, les premiers LIs étaient des chloroaluminates composés de chlorure d'aluminium et de chlorures alcalins. Un exemple de ce type de LIs est AlCl₃-NaCl dont la température de fusion est de 107°C. D'autres générations de LIs ont suivi mais c'est l'utilisation de chloroaluminates associés à des cations imidazolium qui démontrèrent l'intérêt de ces milieux en tant que solvants et catalyseurs.

Utilisés principalement pour la synthèse organique, ce n'est que plus tard que les Lis ont été utilisés comme solvants d'extraction. Si, à ce jour, des dizaines de milliers de publications portant sur les LIs sont recensées, seules quelques centaines portent sur l'extraction de produits naturels.

Bien que les LIs présentent des propriétés intéressantes, ils ne peuvent toutefois pas être considérés comme de véritables solvants « verts ». En effet, leur coût de fabrication ainsi que leur faible biodégradabilité entre autres en font de mauvais candidats.

Les solvants eutectiques profonds (SEPs) constituent une sous-catégorie des liquides ioniques. Les SEPs sont des mélanges d'au moins deux espèces qui s'associent entre elles par des liaisons intermoléculaires non-covalentes. Ces interactions entraînent une diminution énergétique caractérisée par la température de fusion du mélange. On utilise le terme de mélange eutectique lorsque le point de fusion, ou point eutectique, du dit mélange est inférieur à celui des deux composés du mélange pris individuellement (Deep Eutectic Solvents (DESs) and Their Applications. Smith EL, Abbott AP, Ryder KS. Chem. Rev. 2014, 114, 11060-11082).

La synthèse de SEPs se fait par complexation d'un sel d'ammonium quaternaire avec un sel métallique ou une espèce donneuse d'hydrogène. La délocalisation de charge se fait, par exemple, entre un ion halogénure et l'espèce donneuse d'hydrogène résultant ainsi en une diminution du point de fusion du mélange.

Les propriétés des SEPs sont quasi-similaires à celle des Liquides ioniques, leurs avantages étant leur facilité de préparation due, entre autres, au faible coût de leur composé. Parmi eux, on compte le chlorure de choline qui est le composé le plus cité dans la littérature dans la formation de SEPs. Le chlorure de choline permet de former un mélange eutectique avec la plupart des composés donneurs d'hydrogène. Le premier système eutectique en contenant a été formé avec de l'urée.

A partir de ce modèle, nombreux systèmes de mélanges eutectiques ont vu le jour. A ceux-là se sont ajoutés des SEPs mettant en scène d'autres mélanges Cat⁺X⁻. La famille des bétaïnes a donc particulièrement été étudiée pour cette application. Bien que la formation des SEPs soit réalisable avec ces dernières, les mélanges avec le chlorure de choline montrent de meilleurs résultats que ce soit dans la facilité de préparation du solvant ou les rendements d'extraction de molécules d'intérêts. Nam et al. (Green Chem., 2015,17, 1718-1727) ont étudiés plusieurs systèmes de SEPs pour l'extraction végétale de quercétine et de kaempferol. Le chlorure de choline associé à un alcool (glycérol, xylitol) ou un sucre (le glucose) ont été testé en comparaison avec d'autres systèmes tels qu'un mélange bétaine avec un acide carboxylique (l'acide malique). Les résultats de cette étude montrent que l'extraction avec un SEP de type chlorure de choline/xylitol permet d'obtenir en moyenne une concentration 4 fois plus élevée que l'extraction à l'eau.

Toutefois, il faut noter que la viscosité de ces solvants SEP reste un problème pour l'extraction végétale. En effet, certains systèmes requièrent l'ajout d'additif tel que l'eau pour être utilisés. A cela s'ajoute la non-toxicité des SEPs qui n'a pas encore démontré. Les problèmes de biodégradabilité des LIs n'ayant été mis en évidence que plusieurs années après leurs premières utilisations, des études complémentaires doivent être réalisées sur les SEPs avant de pouvoir les considérer comme éco-solvants.

L'équipe du Pr. Verpoorte de l'Université de Leiden a introduit le terme de solvants eutectiques profonds naturels (SEPNa ou NaDES) à partir de 2011. Il s'agirait de mélanges eutectiques composés uniquement de molécules dites naturelles, c'est-à-dire que l'on retrouve au niveau de la cellule végétale US 8632829 B2 décrit un procédé d'extraction de procyanidines de cacao avec des solvants organiques.

Cependant, un besoin constant de solvants d'extraction naturels subsiste.

### RESUME DE L'INVENTION

La présente invention concerne l'utilisation de l'eau de coco comme solvant d'extraction de composés, en particulier pour la préparation de principes actifs, de préférence de principes actifs cosmétiques. L'utilisation de ce liquide naturel permet d'augmenter l'efficacité de l'extraction de certaines molécules, notamment par rapport à l'extraction aqueuse simple.

Ainsi, la présente invention est relative à une utilisation de l'eau de coco comme solvant d'extraction, en particulier pour l'extraction de composés ou de préparation d'extraits à partir de matériel biologique végétal, animal et/ou procaryote.

La présente invention est également relative à une méthode d'extraction de composés ou de préparation d'extraits à partir de matériel biologique végétal, animal et/ou procaryote, comprenant l'incubation dudit matériel biologique végétal, animal et/ou procaryote avec de l'eau de coco, et la récupération des composés ou de l'extrait. De préférence, la méthode comprend une étape d'immersion du matériel biologique dans l'eau de coco; une étape d'extraction du mélange obtenu ; et une étape de récupération des composés ou de l'extrait comprenant une étape de centrifugation et/ou de filtration et/ou d'évaporation et/ou de concentration et/ou de fractionnement et/ou de purification ou une combinaison de ces étapes. En particulier, l'eau de coco et le matériel biologique sont mis en œuvre avec un ratio pondéral compris entre 1 1 et 100 : 1, de préférence entre 2 : 1 et 100 : 1 ou entre 5 : 1 et 20 : 1, par exemple à un ratio de 10 : 1.

Facultativement, un tensioactif anionique, cationique ou non ionique peut être ajouté à l'eau de coco pour faciliter l'extraction des composés.

Facultativement, l'étape d'extraction peut être assistée par microondes ou par utrasons.

Dans un premier mode de réalisation, l'étape d'extraction est réalisée à une température comprise entre 2°C et 100 °C, de préférence entre 20°C et 90°C, par exemple entre 50°C et 80°C pendant 15 minutes à 2 jours, de préférence pendant 30 minutes à 1 jour, et en particulier pendant 1 à 3 heures. Dans un autre mode de réalisation, l'étape d'extraction est réalisée en conditions subcritiques. En particulier, l'extraction est réalisée sous azote et sous pression à des températures comprises entre 100 et 140 °C pendant 5 minutes à 1 heure.

De préférence, le matériel biologique peut être une plante ou une ou des parties de celle-ci.

De préférence, les composés extraits incluent des terpènes, terpènoïdes, flavones and flavonoïdes, stéroïdes, stérols, saponines and sapogénines, alcanes, alcaloïdes, aminés, acides aminés, aldéhydes, irrridoîdes, phénylpropanoïdes, alcools, polyols, lipides, acides gras, lignanes, phénols, pyrones, buténolides, lactones, chalcones, cétones, benzènes, cyclohexanes, glucosides, glycosides, cyanidines, furanes, phorbols, quinones et phloroglucinols sous formes aglycone ou le cas échéant sous forme glycosylée. L'invention peut également être appliquée à l'extraction de molécules bioactives de masse moléculaire élevée telles que des protéines, des peptides, des enzymes, des polysaccharides, des oligosaccharides et des glucides.

Un extrait de matériel biologique végétal, animal et/ou procaryote comprenant une eau de coco est obtenu par la méthode selon la présente invention. Cet extrait est utilisé pour la fabrication d'une composition nutraceutique, d'un produit diététique ou alimentaire, d'un complément nutritionnel, d'une composition pharmaceutique notamment dermatologique ou d'une composition cosmétique ou à une composition nutraceutique, diététique, alimentaire, nutritionnelle, pharmaceutique notamment dermatologique ou cosmétique comprenant cet extrait. Elle est également relative à une méthode de préparation d'une composition nutraceutique, diététique, alimentaire, nutritionnelle, pharmaceutique notamment dermatologique ou cosmétique comprenant la préparation d'un extrait de matériel biologique végétal, animal et/ou procaryote selon la méthode selon l'une quelconque des revendications 2-11 et l'incorporation de l'extrait dans une composition nutraceutique, diététique, alimentaire, nutritionnelle, pharmaceutique notamment dermatologique ou cosmétique.

### DESCRIPTION DETAILLEE DE L'INVENTION

La présente invention concerne l'utilisation de l'eau de coco comme solvant d'extraction L'invention est décrite dans les revendications 1 à 12.

### Eau de coco

Les termes « eau de coco » et « jus de coco » peuvent être utilisés indifféremment dans la présente demande de brevet.

Le cocotier est une plante monocotylédone de la famille des Arecaceae. Il fait partie du genre Cocos dont il n'existe qu'une seule espèce *Cocos nucifera* L.

L'espèce regroupe un grand nombre d'écotypes ou cultivars qui présentent des morphologies et des colorations variées. On distingue généralement les variétés dites « Tall Coconut » (grand cocotier) pouvant croitre en hauteur jusqu'à 20-25 m et des variétés dites « Dwarf Coconut » (Cocotiers Nains) dépassant rarement 12 mètres de hauteur : une troisième catégorie regroupe les hybrides de ces deux variétés. Ainsi, l'eau de coco peut provenir indifféremment d'une variété de grand cocotier, de celle des cocotiers nains ou enfin de celle des hybrides.

La culture du cocotier est largement répandue sous les tropiques. Plus de 80% des surfaces plantées de cocotier se situent en Asie (Inde, Philippines, Indonésie, Sri Lanka, Thaïlande). Le reste se répartit entre l'Afrique, l'Amérique latine, l'Océanie et les Caraïbes.

Le fruit de *Cocos nucifera* L. est une drupe de forme ovoïde ou elliptique, composée d'un épiderme lisse de couleur variable, d'un mésocarpe fibreux (ou bourre), et d'un endocarpe dur lignifié (ou coque) marron foncé contenant une amande qui correspond à l'albumen de la graine. L'amande est creuse et sa cavité centrale contient un liquide stérile appelé « eau de coco » destinée à lui permettre de germer quelles que soient les conditions extérieures.

La noix de coco commercialisée correspond donc au noyau du fruit. L'amande de la noix mûre est consommée telle que ou transformée pour donner l'huile de coco, le lait de coco ou la poudre de noix de coco utilisée en pâtisserie.

Ainsi, par « eau de coco » est désigné le liquide contenu dans la cavité centrale de la noix de coco.

Le fruit de coco atteint la pleine maturité entre 11 et 12 mois après la fécondation. À 5 mois, l'amande commence à former une mince couche de gelée autour de l'intérieur de l'endocarpe. Pendant le processus de maturation, l'amande grossit progressivement et remplace l'eau de coco par des cellules de stockage des lipides. À pleine maturité, l'eau de coco représente entre 15 % et 30% du poids de la noix, la quantité pouvant être récoltée de chaque noix étant d'environ 300 ml mais dépendant notamment de la variété de noix de coco. La composition de l'eau de coco change au fur et à mesure de la maturation.

Dans un premier mode de réalisation, l'eau de coco est une eau de coco du fruit à maturité, c'est-à-dire un fruit récolté entre 11 et 12 mois. Dans un autre mode de réalisation préféré, l'eau de coco est une eau de coco obtenue à partir d'un fruit vert. Par exemple, l'eau de coco est prélevée sur une noix ayant 5 à 11 mois de maturité, par exemple ayant 5, 6, 7, 8, 9 ou 10 mois de maturité, de préférence ayant 6, 7, 8, ou 9 mois de maturité.

Dans un mode de réalisation, l'eau de coco comprend entre 2 et 7 g de matière sèche/ 100 ml.

Dans un mode de réalisation, l'eau de coco comprend entre 1,5 et 7 g de sucres/100ml, dont entre 0,5 et 5 g de sucres réducteurs/100 ml. De préférence, l'eau de coco comprend entre 4 et 6 g de sucres/100ml, dont entre 2 et 4 g de sucres réducteurs/100 ml.

Dans un mode de réalisation, l'eau de coco comprend entre 0,75 et 2,5 g d'acides aminés/100 ml, de préférence entre 1 et 2 g/100 ml.

Dans un mode de réalisation, l'eau de coco présente une acidité comprise entre 0,5 et 2 meq/100 ml, de préférence entre 0,7 et 1 meq/100 ml.

Ainsi, dans un mode de réalisation particulier, l'eau de coco est caractérisée par la combinaison de 2, 3, ou 4 caractéristiques parmi les suivantes :
- entre 2 et 7 g de matière sèche/ 100 ml ;
- entre 1,5 et 7 g de sucres/100ml, dont de préférence entre 1 et 5 g de sucres réducteurs/100 ml ;
- entre 0,75 et 2,5 g d'acides aminés/100 ml ; et
- une acidité comprise entre 0,5 et 2 meq/100 ml, de préférence entre 0,7 et 1 meq/100 ml.

De préférence, les sucres sont choisis parmi le sucrose, le glucose, le fructose, le galactose, le xylose et le mannose.

L'eau de coco comprend également des acides aminés.

De nos jours l'eau de coco constitue une boisson tropicale rafraîchissante dont le marché international est en pleine expansion. Elle est également décrite comme une « boisson de réhydratation pour sportif». En conséquence, il existe de nombreuses sources d'eau de coco disponibles commercialement.

### Procédés ou méthodes d'extraction

La présente invention est relative à l'utilisation de l'eau de coco comme solvant d'extraction, en particulier pour l'extraction de substances à partir de matériel biologique végétal et/ou animal et/ou procaryote.

Elle est également relative à un procédé ou méthode d'extraction de composés ou de préparation d'extraits à partir de matériel biologique végétal, animal et/ou procaryote, comprenant la mise en contact dudit matériel biologique végétal, animal et/ou procaryote avec de l'eau de coco, et la récupération des composés ou de l'extrait. Les procédés d'extraction sont bien connus de l'homme du métier.

Plus particulièrement, le procédé peut comprendre une étape d'immersion du matériel biologique dans l'eau de coco; une étape d'extraction, avec ou sans agitation, comme par exemple de macération ou décoction ou percolation ou infusion du mélange obtenu ; éventuellement une étape de centrifugation et/ou de filtration et/ou d'évaporation et/ou de concentration et/ou de fractionnement et/ou de purification ou une combinaison de ces étapes.

Dans un mode de réalisation particulier, un tensioactif anionique, cationique ou non ionique peut être ajouté à l'eau de coco pour faciliter l'extraction des composés cibles.

Lors de l'étape de mise en contact ou d'immersion, l'eau de coco et le matériel biologique sont mis en œuvre avec un ratio en poids compris entre 1: 1 et 100:1, de préférence entre 2: 1 et 100 : 1 ou entre 5 : 1 et 20 : 1, par exemple à un ratio de 10 : 1. Le procédé peut comprendre une étape d'ajustement du pH d'extraction qui peut être compris entre 3 et 10. Le pH de l'extrait final peut être ensuite ajusté le cas échéant à un pH proche de la neutralité Les conditions de température pour l'étape de macération ou percolation ou infusion du mélange sont adaptées suivant le matériel biologique de départ et la substance à extraire ou l'extrait à préparer. Par exemple, la température peut être comprise entre 2°C et 100 °C, de préférence entre 20°C et 90°C, par exemple entre 50°C et 80°C.

La durée de l'étape de de macération ou percolation ou infusion est également à adapter suivant le matériel biologique de départ et la substance à extraire ou l'extrait à préparer. La durée dépend également de la méthode d'extraction mise en œuvre.

Par exemple, la durée peut être comprise entre 15 minutes et 2 jours, de préférence entre 30 minutes et 1 jour, et en particulier entre 1 heure et 3 heures. Ainsi, la durée peut être 30 minutes, 1 heure, 2 heures, ou 3 heures.

L'étape d'extraction peut être réalisée sous agitation ou sans, avec ou sans sonication, éventuellement sous radiation (infrarouge, microonde), sous pression ou en conditions atmosphériques. Ainsi, l'étape d'extraction peut être assistée par microondes ou par utrasons.

De préférence, l'extraction est réalisée en conditions atmosphériques.

Cependant, l'eau de coco peut également être mise en œuvre dans des techniques d'extraction en eau subcritique. L'extraction est réalisée dans un réacteur à microondes à haute performance. Cette extraction est de préférence réalisée sous azote et sous pression, par exemple de 30 bars. L'extraction peut par exemple être faite à des températures comprises entre 100 et 140 °C, de préférence entre 105°C et 130°C, et pendant 5 minutes à 1 heure, de préférence entre 10 minutes et 30 minutes.

L'extraction peut être faite en une passe ou plusieurs passes. De préférence, l'extraction est réalisée en une passe.

Le procédé résulte en la préparation d'un extrait de matériel biologique. L'extrait obtenu par le procédé constitue un des objets de la présente invention.

Cependant, le procédé peut comprendre une ou plusieurs étapes ultérieures de purification d'une substance contenu dans l'extrait de matériel biologique. De manière alternative, l'extrait de matériel biologique peut être utilisé directement pour la fabrication d'une composition nutraceutique, d'un produit diététique ou alimentaire, d'un complément nutritionnel, d'une composition pharmaceutique notamment dermatologique ou d'une composition cosmétique sans étape de purification.

En particulier, selon un mode particulièrement avantageux, le procédé ne comprend pas l'ajout de solvant organique.

### Matériel biologique végétal, animal et/ou procaryote

L'eau de coco est donc utile pour extraire des composés ou préparer des extraits à partir de matériel biologique végétal et/ou animal et/ou procaryote. Le matériel végétal peut être terrestre ou aquatique.

Par exemple, le matériel biologique peut être une plante ou une ou des parties de celle-ci ; un animal ou une ou des parties de celui-ci ; une algue, un lichen, un champignon, une levure, une moisissure, ou une bactérie. Par exemple, la ou les parties de plantes peuvent être choisies parmi le bois, les racines, le rhizome, l'écorce, les fleurs, les pétales, les sépales, les graines, les fruits, les parties aériennes, en particulier la tige, les feuilles. Le matériel végétal utilisé peut être différentié ou non différentié (cals, cellules souches végétales).

Le matériel biologique peut subir des traitements préalables à l'extraction. Le matériel biologique peut être préalablement séché. Par exemple, le matériel biologique peut être broyé, coupé ou autre. Le broyage peut être réalisé à l'aide d'un broyeur à billes, broyage au mortier, broyage aux ultrasons, broyage à l'aide d'un mixeur, etc.

Par exemple, le matériel biologique peut être choisi parmi la liste non-exhaustive suivante :
*Agave atrovirens*, *Malpighia emarginata, Aucoumea klaineana*, *Cladosiphon okamuranus*, *Macadamia ternifolia, Callitris introtropica*, *Illicium verum, Orbignya oleifera, babassu, Haberlea rhodopensis, Morinda officinalis, Ilex paraguariensis, Borojoa patinoi, Pichia, Bertholletia excelsa, Ptychopetalum olacoides, Paullinia cupana, Caryocar brasiliense, Copernicia cerifera, Dolichos lablab, Trifolium repens, Limnanthes alba, Meadowfoam estolide, Syringa oblata affinis, Amomum kravanh, , Angelica polymorpha*, *Viscum album, Lithospermum officinale*, *Rhinacanthus communis*, *Lilium candidum, Patrinia villosa*, *Anthemis nobilis*, *Peucedanum praeruptorum*, *Hedyotis diffusa*, *Oldenlandia diffusa, Mucuna birdiana, Betula alba, Bletilla striata, Plumeria alba, Beauveria bassiana , Fraxinus chinensis, Michelia alba, Pyrus bretschneideri, Quercus alba, Ampelopsis japonica, Salix alba, Hydrastis canadensis, Imperata cylindrica,*, *Aquilaria sinensis*, *Inula cappa*, *Melaleuca Leucadendra, Cynanchum stauntonii, Atractyloidesmacrocephala, Nymphaea alba, Tuber magnatum , Perilla frutescens, Pulsatilla chinensis*, *Cynanchum atratum*, *Dictamnus dasycarpus, Helichrysum angustifolium, Lupinus albus, Iris pallida, Lilium brownii, Centaurium erythraea, Thymus serpyllum mongolicus, Lotus corniculatus*, *Rosa centifolia*, *Fagraea berteroana, Platycladus orientalis,*, *Orchis maculata, Geranium maculatum*, *Tabebuia impetiginosa*, *Scutellaria barbota*, *Cyathea cumingii, Brassica oleracea gemmifera*, *Mentha arvensis, Mentha haplocalix, Artemisia vulgaris, Atractyloides chinensis, Laminaria hyperborea*, *Cedrus atlantica*, *Liriodendron tulipifera, Sequoia sempervirens, Hamamelis virginiana, Pinus strobus*, *Juniperus virginiana*, *Schizandra chinensis*, *Scrophularia buergeriana, Hovenia dulcis, Leptospermum petersonii, Ficus pumila*, *Cupressus funebris, Dolichos lablab*, *Prinsepia utilis, Enteromorpha compressa, Astragalus complanatus, Citrus depressa peel, Polygonum aviculare, Vanilla planifolia, Iris versicolor, Angelica keiskei, Cakile maritima, Eryngium maritimum, Artemisia scoparia, Cetraria islandica, Lens esculenta, Agaricus blazeii, Peumus boldus , Rheum undulatum, Borago officinalis*, *Spinacia oleracea*, *Ananas sativus*, *Sclerocarya birrea, Spirulina platensis*, *Haematoxylon campechianum*, *Phaseolus vulgaris*, *Elaphe carinata*, *Cynara scolymus*, *Pheretima aspergillum*,, *Echinacea pallida*, *Xanthium sibiricum*, *Atractyloides lancea, Acorus calamus, Crocus sativus, Avena strigosa, Alpinia katsumadai, Amomum tsao-ko, Hedychium spicatum, Salicornia herbacea, Fragaria ananassa, Saxifraga sarmentosa, Arbutus unedo, Gossypium herbaceum*, *Melilotus officinalis, Sarcandra glabra*, *Biota orientalis*, *Platycladus orientalis*, *Thuja orientalis, Baccharis genistelloides*, *Jania rubens, Camellia sinensis*, *Aphloia theiformis*, *Bupleurum chinensis, Acorus calamus, Hedera nepalensis sinensis, Pulsatilla koreana, Angelica gigas, Epimedium koreanum*, *Plantago asiatica*, *Echium plantagineum*, *Aquilaria agallocha*, *Tamarix chinensis, Corydalis turtschaninovii, Boswellia serrata, Nymphaea lotus, Fucus serratus, Phaseolus angularis, Ganoderma neo-japonicum, Pinus densiflora, Phaseolus calcaratus, Ganoderma lucidum, Cassia alata , Passiflora alata, Ailanthus altissima, Lactuca virosa, Bellis perennis*, *Paeonia veitchii, Codonopsis tangshen*, *Coix lacryma-jobi ma-yuen*, *Phellodendron chinense*, *Zanthoxylum piasezkii, Vladimiria souliei, Cyathula officinalis, Ligusticum chuanxiong, Dipsacus asper, Andrographis paniculata, Sedum sarmentosum*, *Betula pendula*, *Ulmus davidiana*, *Brasenia schreberi, Mentha pulegium*, *Argania spinosa, Juniperus formosana, Asparagopsis armata, Robinia pseudoacacia, Rosa roxburghii, Actinidia Chinensis sitosa*, *Capparis spinosa*, *Codium fragile*, *Sterculia urens*, *Acanthopanax senticosus, Eleutherococcus senticosus, Caesalpinia spinosa, Cnicus benedictus, Mitracarpus scaber, Hydrangea serrata, Plantago major, Alpinia galanga, Passiflora quadrangularis, Vaccinium macrocarpon, Citrus tangerina, Rhodiola crenulata, Theobroma grandiflorum, Portulaca grandiflora, Sesbania grandiflora , Cereus grandiflorus, Rosa odorata gigantea, Lagerstroemia speciosa, Cirsium japonicum, Grindelia robusta, Musa sapientum, Pentaclethra macroloba, Cannabis sativa, Hibiscus cannabinus, Hordeum vulgare, Cimicifuga heracleifolia*, *Allium sativum*, *Sinocalamus beecheyanus pubescens, Sargentodoxa cuneata*, *Plantago lanceolata*, *Uncaria macrophylla*, *Sargassum pallidum*, *Zostera marina*, *Buddleja davidii*, *Zizyphus jujuba*, *Artemisia sieversiana*, *Anigozanthos flavidus*, *Salvia miltiorrhiza, Cimicifuga simplex, Vitex trifolia simplicifolia, Crataegus monogina, Lessonia nigrescens , Lophatherum gracile, Angelica sinensis*, *Codonopsis pilosula*, *Canavalia gladiata*, *Cardiospermum halicacabum, Garcinia mangostana, Oryza sativa*,, *Iris germanica*, *Cornus controversa*, *Celastrus paniculata, Thamnolia vermicularis*, *Kochia scoparia*, *Lycium chinense*, *Ficus tikoua*, *Rehmannia glutinosa, Voandzeia subterranea, lichen, Poterium sanguisorba, Sanguisorba officinalis, Cupressus sempervirens, Polygonatum kingianum*, *Daphne feddei, Camellia reticulata, Selaginella pulvinata, Clitoria ternatea*, *Eugenia caryophyllus, Taxus cuspidata*, *Viola mandshurica*, *Angelica acutiloba, Typha orientalis, Aloe perryi, Prunus yedoensis, Cordyceps sinensis mycelium*, *Benincasa cerifera, Benincasa hispida*, *Malva verticillata* , *Origanum heracleoticum*, *Berberis aquifolium*, *Nasturtium officinale, Pachyrrhizus erosus*, *Angelica pubescens*,, *Vaccinium uliginosum, Eucommia ulmoides, Lamium album, Yucca brevifolia, Ona tuberosa, Sargassum muticum, Cassia obtusifolia, Polygonatum multiflorum*, *Haslea ostrearia*, *Ormenis multicaulis*, *Tupidanthus calyptratus*, *Eysenhardtia polystachya, Anthriscus sylvestris, Centipeda minima, Potentilla anserina, Persea gratissima, Acacia catechu, Uncaria gambir, Cynamchum auriculatum, zymomonasferment, Terminalia ferdinandiana , Helichrysum stoechas, Rosa gallica, Lavandula stoechas, Crocus sativus, Anona squamosa, Carica papaya*, *Lycopersicon esculentum, Solanum lycopersicum, Psidium guajava, Cassia senna, Stellaria média*, *Ledebouriella divaricata*, *Saposhnikovia divaricata*, *Lilium candidum*, *Kigelia africana, Aframomum melegueta, Prunus africana, Khaya senegalensis, Saponaria officinalis, Torreya grandis, Dioscorea hypoglauca*, *Stephania tetrandra*, *Passiflora incarnata*, *Padina pavonica, Clinopodium chinense, Hyacinthus orientalis, honey, royal jelly, Pteris multifida, Impatiens balsamina, Yucca vera , Citrus medica sarcodactylis, Sechium edule, Epilobium fleischeri, Poria cocos, Spirodela polyrrhiza, Chlorella emersonii, Tetraselmis suecica*, *Plankton, Pinus radiata*, *Citrus tangerina*, *Rubus chingii, Rubus idaeus, Glycyrrhiza uralensis, Pueraria thomsonii, Chrysanthemum boreale, Dendranthema lavandulifolium*, *Origanum majorana*, *Ipomoea batatas*, *Nardostachys chinensis*, *Saccharum officinarum*, *citrus Nobilis ponki, Citrus reticulata, Canarium album, Polygonum perfoliatum, Alpinia officinarum, Scutellaria alpina*, *Leontopodium alpinum, Rhododendron ferrugineum, Linum alpinum , Ligusticum sinense*, *Pueraria lobata*, *Carum carvi*, *Actinidia polygama*, *rhizobian*, *Pelvetia canaliculata, Hypnea musciformis, Cibotium barometz, Rosa canina, Ilex cornuta, Poncirus trifoliata , Lycium chinense*, *Broussonetia papyrifera, Copaifera officinalis resin, Cryptolepis buchananii, Ferula galbaniflua, Eriocaulon buergarianum*, *Polygala japonica*, *Atractyloides japonica*, *Hypericum perforatum, Cyrtomium fortunei, Glycyrrhiza glabra*, *Anogeissus leiocarpus, Malpighia glabra*, *Usnea barbota glabrescens*, *Cola nitida*, *Chaenomeles sinensis*, *Pogostemon cablin*, *Desmodium styracifolium, Dioscoreae persimilis, Curcuma kwangsiensis, Citrus nobilis, Choerospondias axillaris,, Osmanthus fragrans, Phyllostachis bambusoides, Entada phaseoloides, Vernonia cumingiana, Pinus pinaster, Morinda citrifolia, Laminaria japonica, Crambe maritima, Adenanthera pavonina, Crithmum maritimum*, *Lygodium japonicum,*, *Amomum longiligulare*, *Pancratium maritimum*, *Ximenia americana*, *Calophyllum inophyllum*, *Cladosiphon novae-caledoniae, Phoenix dactylifera*, *Davallia mariesii, Anastatica hierochuntica, Cassia mimosoides, Tropaeolum majus, Apium graveolens*, *Aloe ferox*, *Terminalia chebula*, *Albizia julibrissin*, *Polygonum multiflorum*, *Magnolia grandiflora, Phyllacantha fibrosa*, *Tuber melanosporum*, *Ribes nigrum*, *Juglans nigra*, *Kadsura coccinea, Ganoderma atrum*, *Salix nigra*, *Secale cereale*, *Rubus fruticosus*, *Morus nigra*, *Populus nigra, Prunus serotina*, *Diospyros lotus*, *Sesamum indicum, Trifolium pratense, Ribes rubrum, Alpinia galanga, Coccinia indica, Eugenia uniflora, Carthamus tinctorius*, *Chrysanthemum coccineum, Pyrola incarnata*, *Acer rubrum*, *Plumeria rubra*, *Rhodiola rosea*, *Narcissus poeticus*, *Ononis spinosa, Nephelium lappaceum, Oxycoccus palustris*, *Bixa orellana*, *Spergularia rubra*, *Zingiber zerumbet, Pinus koraiensis*, *Lonicera hypoglauca*, *Freesia armstrongii*, *Malpighia punicifolia*, *Delesseria sanguinea, Adansonia digitata, Magnolia officinalis, Eriodictyon crassifolium, Bergenia crassifolia, Picrorhiza scrophulariflora, Piper nigrum*, *Trigonella foenum-graecum*, *Daucus carota sativa, Juglans regia, Juglans mandshurica*, *Lespedeza bicolor, Cucurbitaceae, Viscum coloratum*, *Drynaria fortunei , Phalaenopsis amabilis, Dendrobium phalaenopsis, Saxifraga stolonifera, Cymbidium grandiflorum, Polygonum cuspidatum*, *Melaleuca alternifolia, Fraxinus ornus*, *pollen*, *Zanthoxylum bungeanum, Pterocarpus marsupium*, *Arachis hypogaea*, *Brassica oleracea botrytis*, *Rubus chingii*, *Uncaria sinensis, Taraxacum sinicum*, *Alpinia chinensis, Fuscoporia obliqua sclerotium, Inonotus obliquus, Sophora japonica*, *Dendrobium loddigesii*, *Phellodendron amurense*, *Dendrobium chrysanthum, Cucumis sativus*, *Mortierella, Patrinia scabiosaefolia, Fritillaria verticillata bulb, Artemisia annua, Primula veris, Clintonia borealis, Peucedanum graveolens*, *Vitex negundo*, *Hibiscus abelmoschus, Michelia champaca, Coptis chinensis stalk, Gentiana lutea, Phellodendron chinense, Astragalosides, Engelhardtia chrysolepis*, *Scutellaria baicalensis, Dioscorea panthaica*, *Narcissus pseudo-narcissus, Boerhavia diffusa, Lupinus luteus, Heteropanax fragrans*, *Smilax aristolochiaefolia, Cistus incanus, Vladimiria souliei cinerea*, *Tephrosia purpurea*, *Grifola frondosa, Pimpinella anisum*, *Foeniculum vulgare*, *Anetholea anisata, Glechoma longituba, Kniphofia uvaria, Pyracantha fortuneana, Cannabis sativa, Gnaphalium leontopodium, Simmondsia chinensis*, *Agastache rugosa, Ageratum conyzoides , Passiflora edulis, Abrux cantoniensis*, *Celosia cristata*, *Cinchona succirubra*, *Morus bombycis, Paederia scandens*, *Polygonatum sibiricum*, *Spatholobus suberectus*, *Kummerowia striata*, *Acer palmatum, Centella asiatica*, *Larrea divaricata, Spirulina maxima, Chlorella minutissima, Angelica tenuissima*, *Tribulus terrestris*, *Bulnesia sarmientoi*, *Panicum miliaceum*, *Cirsium*, *Irvingia gabonensis, Lepidium sativum*, *Ulmus campestris, Hibiscus militaris*, *Tasmannia lanceolata, Pseudoalteromonas*, *Bacopa monniera*, *Ruscus aculeatus*, *Magnolia acuminata*, *Myrocarpus fastigiatus, Fraxinus szaboana, Gentiana rigescens, Garcinia cambogia, Taraxacum sinicum, Agave rigida*, *Citrus hystrix, Epimedium sagittatum, zingiber officinale*, *Hedychium coronarium , Curcuma longa*, *Polygonatum cyrtonema, Bombyx mori, Dalbergia odorifera, Astragalus mifer, Liquidambar styraciflua, Gynoma pentaphyllum, yeast, Sambucus nigra, Goodyera kwangtungensis, Dendrobium nobile*, *Cuscuta japonica*, *Chrysanthellum indicum*, *Phaseolus lunatus*, *Cibotium barometz, Spilanthes acmella*, *Lysimachia christinae*, *Pseudolarix kamepferi*, *Sarothamnus scoparius, Sciadopitys verticillata*, *Stephania cepharantha*, *Rosa laevigata*, *Calendula officinalis*, *Thuja occidentalis*, *Physalis alkekengi Franchetii*, *Malva sinensis*, *Ulex europaeus*, *Nepeta cataria*, *Schizonepeta tenuifolia*, *Pilea salwinensis, Murraya exotica*, *Allium tuberosum*, *Platycodon grandiflorum*, *Tanacetum vulgare*, *Chrysanthemum morifolium*, *Cichorium intybus*, *Polymnia sonchifolia, Dioscorea composita, Citrus reticulata, Equisetum giganteum, Sequoiadendron gigantea , Nymphaea gigantea, Macrocystis pyrifera*, *Lithothamnium calcarum*, *Pinus pentaphylla*, *Serenoa serrulata, Symphytum officinale, Selaginella tamariscina, Lilium tigrinum, Brassica oleracea capitata , Terminalia sericea*, *cassia Obtusifolia*, *Diospyros lotus*, *Kohhii ekisu, Abelmoschus esculentus, Hibiscus esculentus, Arnica chamissonis, Kunzea ericoides, Zingiber cassumunar, Piper methysticum , Dianthus caryophyllus*, *Theobroma cacao*, *Colax jugosus*, *Citrus clementina*, *Kluyveromyces, Laminaria cloustoni, Agarum cribosum*, *cuminum cyminum*, *Prunus amygdalus amara*, *Sophora angustifolia*, *Citrus aurantium amara, Momordica charantia*, *Fraxinus rhynchophylla*, *Picrasma quassioides*, *Carapa guaianensis*, *Citrus aurantium currassuviensis*, *Aloe barbadensis*, *Tinospora sinensis* , *Notopterygium forbesii*, *Lavandula intermedia*, *Tussilago farfara*, *fukitanpopo ekisu, Tussilago farfara* , *Scutellaria galericulata, Sasa quelpaertensis, Artemisia princeps, Ecklonia kurome , Chenopodium quinoa, Trichosanthes kirilowii, Tilia platyphyllos , Liriope muscari, Mahonia bealei, rahnellasoy, Cassia fistula* , *Myrica cerifera*, *Helichrysum bracteatum* , *Myrica cerifera*, *Mentha piperita, Cochlearia armoracia* , *Capsicum annuum, Polygonum hydropiper, Moringa oleifera, Citrus aurantifolia, Raphanus sativus, Eucalyptus globulus* , *Lonicera caerulea* , *Terminalia catappa* , *Bidens tripartita* , *Geranium wilfordii* , *Omphalia lapidescens* , *Prunus salicina* , *Eclipta prostrata* , *Litchi chinensis, Forsythia suspensa, Nelumbium speciosum, Nelumbo nucifera, Cymbidium lianpan, Eperua falcata, Lansium domesticum, Mesona chinensis, Zanthoxylum nitidum, Sorghum bicolor, Hylocereus undatus* , *Aralia elata, Ligusticum jeholense* , *Anthyllis vulneraria* , *Polygonum tinctorium* , *Phellinus linteus, Ipomoea hederacea, Schizosaccharomyces, Cymbopogon schoenanthus, Lysimachia foenum-graecum, Campsis grandiflora, Hedera rhombea, Ocimum basilicum, Mentha spicata, Mentha viridis , Epilobium angustifolium* , *Cryptomeria japonica* , *Serissa serissoides* , *Gentiana scabra*, *Artemisia dracunculus* , *Agave americana* , *Bambusa vulgaris* , *Euphoria longan*, *Nephelium longana, Dimocarpus longan, Piper betle* , *Rhaponticum uniflorum*, *Phragmites communis, Aloe vera, Aloe matsu ekisu, Visnaga vera, Yucca aloifolia*, *Phragmites communis* , *Arundo donax* , *Artemia* , *Gossypium hirsutum* , *Pyrola calliantha* , *Liquidambar formosana*, *Plantago ovata* , *Adenophora retraphylla* , *Phalaenopsis lobbi*, *Apocynum venetum* , *Pikea robusta*, *Coffea robusta*, *Thujopsis dolabrata branch* , *Momordica grosvenori, podocarpus macrophyllus*, *ocimum Basilicum*, *Balanites roxburghii, Raphanus sativus* , *Metaplexis japonica, Trachelospermum jasminoides, Camellia kissii, Kalanchoe pinnata* , *Arachis hypogaea* , *Phaseolus mungo* , *Phaseolus radiatus* , *Vigna radiata , Melaleuca leucadendron viridiflora*, *Melaleuca viridiflora*, *Enantia chlorantha* , *Acacia decurrens , Amomum villosum xanthioides* , *Hieracium pilosella* , *Humulus japonicus* , *Gnetum parvifolium, Gentiana straminea* , *Quercus acutissima* , *Verbena officinalis* , *Dendrobium fimbriatum oculatum , Portulaca oleracea* , *Harungana madagascariensis* , *baphicacanthvs cusia, Romneya coulteri, Zantedeschia aethiopica* , *Pinus massoniana* , *Lantana camara*, *Lepidium meyenii,, Ophiopogon japonicus, Elaeagnus glabra, Vitex trifolia, Cynanchum versicolor, Ona japonica, Lonicera caprifolium , Mangifera indica* , *Erodium stephanianum* , *Sapindus rarak, Siegesbeckia glabrescens*, *Solidago virgaurea* , *Terminalia bellerica* , *Coleus forskohlii*, *Plectranthus barbatus* , *Lonicera dasystyla , Chrysanthemum sinense* , *Cinchona pubescens* , *Phyllostachys nigra* , *Gouania javanica* , *Paulownia tomentosa* , *Verbascum thapsus* , *Mauritia flexuosa* , *Physalis pubescens* , *Lindera latifolia* , *Anona cherimolia*, *emmeisou ekisu, Corylus mendshurica* , *Atractyloides lancea*, *Melothria heterophylla, Rubus parvifolius, Hierochloe odorata* , *Commiphora myrrha* , *Cymbopogon martini, Sedum rosea* , *Rosa rugosa* , *rose petal, Aniba rosaeodora* , *Hibiscus sabdariffa*, *Epilobium roseum* , *Prunus mume , Monarda didyma* , *Carya illinoinensis* , *Orbignya speciosa* , *Acacia concinna* , *Ahnfeltia concinna , Actinidia deliciosa* , *Rubus deliciosus* , *Polygala senega* , *Sambucus canadensis, Campsis radicans , Corylus americana*, *Astragalus membranaceus mongholicus, Rosmarinus officinalis* , *Aglaia odorata , Krameria triondra, Myroxylon pereirae* , *Buddleja officinalis, Melicope hayesii, Polysiphonia lanosa , Taraktogenos kurzii*, *leuconostocradish* , *Changium smyrnioides* , *Astragalus membranaceus , Ormenis mixta, moroccan lava clay, Jasminum sambac, Eclipta prostrata, Fucus vesiculosus, Juniperus mexicana* , *Fouqueria splendens*, *Larrea mexicana* , *Dioscorea mexicana* , *Eugenia caryophyllata , Chamomilla recutita* , *Matricaria chamomilla*, *Paeonia suffruticosa* , *Vitex negundo cannabifolia , Momordica cochinchinensis, Hibiscus mutabilis, Chaenomeles sinensis, Oroxylum indicum* , *Hibiscus syriacus, Magnolia obovata, Indigofera tinctoria, Aloe arborescens, Astrocaryum murumuru, Bombax malabaricum, Limonia acidissima, Akebia quinata, Aucklandia lappa, Equisetum hiemale, Cuscuta australis*, *Harpagophytum procumbens* , *Durvillea antartica* , *Marsdenia condurango* , *Pfaffia paniculata* , *Artemisia abrotanum*, *Choerospondias axillaris* , *Schisandra sphenanthera* , *Sapindus emarginatus, Phyllostachys pubescens* , *endomyces, Arnebia guttata, Arabidopsis thaliana, Gellidiela acerosa* , *Lycium barbarum*, *Citrus limon, Eucalyptus citriodora*, *Backhousia citriodora* , *Thymus citriodorus, Lippia citriodora* , *Cymbopogon citratus, Arctium lappa*, *Rhododendron chrysanthum , Rabdosia ternifolia, Achyranthes bidentata, Butyrospermum parkii (shea butter), Origanum vulgare, Opuntia streptacantha* , *Picea excelsa, Clematis apiifolia, Ligustrum lucidum*, *Solanum dulcamara, Thymus serpyllum* , *Juniperus communis* , *Salvia sclarea* , *Levisticum officinale* , *Syringa vulgaris , Glechoma hederacea* , *Malva sylvestris* , *Prunus humilis* , *Cystoseira tamariscifolia*, *Taraxacum officinale* , *Peucedanum ostruthium, Rubia tinctorum, Carum petroselinum* , *Petroselinum sativum , Achillea millefolium*, *Frangula alnus* , *Marrubium vulgare* , *Urtica urens* , *Nuphar luteum*, *Inula britannica* , *Primula vulgaris* , *Fraxinus excelsior* , *Xanthium strumarium* , *Pinus sylvestris* , *Ribes grossularia* , *Lapsana communis* , *Tilia cordata* , *Sorbus aucuparia* , *Abies alba, Prunus domestica , Castanea sativa* , *Dryopteris filix-mas, Agrimonia eupatoria, Larix europaea, Aesculus hippocastanum , Philadelphus coronarius* , *Populus tremuloides* , *Cypripedium pubescens* , *Lycopodium clavatum , Quercus suber, Fagus sylvatica, Isatis tinctoria, Prunus cerasus* , *Prunus avium* , *Berberis vulgaris, Brassica napus* , *Vaccinium myrtillus* , *Corylus avellana bud, Ascophyllum nodosum* , *Eupatorium fortunei, Curcuma phaeocaulis, Eriobotrya japonica, Terminalia bellerica, Humulus lupulus strobile, Saccharomyces cerevisiae, Plantago depressa, Psalliota campestris* , *Gaultheria procumbens* , *Malus domestica cell culture, Malus pumila, Pyrus malus* , *Rhamnus purshiana, Ficus religiosa, Vitis vinifera , Clematis vitalba, Citrus paradisi* , *Taraxacum mongolicum* , *Syzygium jambos* , *Limonium vulgare, Tilia vulgaris* , *Pyrola decorata* , *Sargassum vulgare* , *Citrus medica vulgaris, Triticum aestivum , Aesculus chinensis* , *Paris polyphylla chinensis* , *Alnus firmifolia* , *Rhus verniciflua, Thaumatococcus danielli, Capsella bursa-pastoris, Porphyra umbilicalis, Daemonorops draco, Epipremnum pinnatum* , *Sasa kurilensis, Lythrum salicaria, Acmella oleracea, Euryale ferox, Rubia cordifolia, Notopterygium incisum, Ecklonia cava, orchis mascula, Rosa multiflora, Polygonum fagopyrum, Solanum melongena , Apium graveolens*, *Gentiana macrophylla*, *Zanthoxylum piperitum, Prinsepia utilis*, *Bambusa tuldoides munro, Artemisia annua,, Helwingia japonica, Vatica astrotricha, Bambusa textilis, Celosia argentea* , *Swertia mileensis* , *Dianthus superbus, Aspergillus ferment, Rhodiola sacra* , *Macadamia integrifolia*, *Polygonum bistorta*,, *Undaria pinnatifida*, *Coleus barbatus* , *Panax ginseng* , *Moschus artifactus, Lonicera japonica, Lilium japonicum, Lotus japonicus symbiosome, Chamaecyparis obtusa , Cnidium officinale* , *Angelica japonica* , *Torreya nucifera* , *Coptis japonica* , *Castanea crenata , Chaenomeles japonica, Nuphar japonicum, Dioscorea japonica, Prunus lannesiana, Swertia japonica , Callicarpa japonica, Rubus villosus* , *Uncaria tomentosa, Terminalia chebula tomentella, Epimedium pubescens* , *Yucca filamentosa*, *Cystoseira amentacea, Caespitosa branchycarpa* , *Cistanche deserticola, Myristica fragrans* , *Cinnamomum cassia, Furcellaria lumbricalis* , *lactococcus ferment, Boswellia carterii*, *Thymus mastichina* , *Actinidia arguta* , *Prinsepia uniflora serrata* , *Crataegus oxyacantha, Saururus chinensis, Gleditsia triacanthos, Gentiana triflora, Sparganium stoloniferum, Viola tricolor, Bidens pilosa, Akebia trifoliata, Sapindus trifoliatus, Epimedium sagittatum* , *Larrea tridentata* , *Artemisia umbelliformis*, *Chimaphila umbellata* , *Morus alba* , *Taxillus chinensis* , *Leptospermum scoparium* , *Adenophora stricta*, *Ammopiptanthus mongolicus* , *Hippophae rhamnoides, Adenium obesum, Abronia villosa, Helichrysum arenarium, Barosma betulina, Camellia japonica* , *Artemisia montana* , *Litsea cubeba* , *Alpinia japonica* , *Arnica montana* , *Pyrus sorbus, Crataegus pinnatifida major, Scabiosa arvensis, Liriope spicata prolifera, Kaempferia galanga, Prunus davidiana, Ipomoea hungaiensis, Hyptis suaveolens, Dioscorea opposita,, Lonicera confusa, Wasabia japonica* , *Cornus officinalis* , *Caulerpa taxifolia*, *Corallina officinalis* , *Paeonia lactiflora* , *Bergenia ligulata, Cnidium monnieri, Fragaria indica, Waltheria indica, Periostracum serpentis, Belamcanda chinensis, Thymus vulgaris* , *Rosa moschata, Lycopodium japonicum, Himanthalia elongata, Crambe abyssinica, Hyssopus officinalis, Orthosiphon stamineus, Cimicifuga foetida, Rhodiola sacra, Ocimum sanctum* , *Acorus tatarinowii, Ulva lactuca*, *asparagus officinalis* , *Lysionotus pauciforus* , *Gypsum fibrosum, Centipeda cunninghamii* , *Aleurites moluccanus bakoly* , *Punica granatum* , *Melaleuca ericifolia*, *Dianthus chinensis* , *Centaurea cyanus, Quisqualis indica* , *Diospyros kaki, Streptococcus thermophilus ferment, Thermus thermophillus ferment, Gymnema sylvestre, Nardostachys jatamansi , Polygonum multiflorum*,, *Euterpe oleracea* , *Althaea officinalis* , *Althaea rosea* , *Salvia japonica , Salvia officinalis* , *Dioscorea batatas, Evernia furfuracea* , *Rhododendron arboreum*, *trichosanthes rosthornii* , *Mentha aquatica* , *Silybum marianum* , *Polygonum orientale*, *Pongamia pinnata , Aphanothece sacrum, Sisymbrium irio, Cardamine lyrata, Narcissus tazetta bulb, Typha angustifolia, Menyanthes trifoliata*, *Nymphaea tetragona* , *Withania somnifera*, *Luffa cylindrica* , *Tricholoma matsutake, Armillaria matsutake, isatis indigotica* , *Cola acuminata* , *Cola acuminata* , *Liquidambar orientalis, Caesalpinia sappan, Jasminum officinale* , *Jasminum grandiflorum, Setaria italica, Citrus aurantium tachibana, Juniperus oxycedrus* , *Tamarindus indica* , *Physalis alkekengi, Polygonum capitatum, Ziziphus jujuba spinosa, Allium sativum* , *Cyathea medullaris, Vitex agnus castus, Fusanus spicatus* , *Lavandula spica, Cucurbita maxima* , *Shorea robusta resin, Amomum xanthioides , Cynomorium songaricum, Vanilla tahitensis, Gardenia tahitensis, Agave tequilana, Pseudostellaria heterophylla* , *Pueraria mirifica*, *Epiphyllum oxpetalum*, *Santalum album* , *Rheum tanguticum , Laminaria saccharina, Acer saccharum* , *Pinus lambertiana, Prunus persica* , *Rhodomyrtus tomentosa , Turnera diffusa*, *Ampelopsis cantoniensis grossedentata* , *Parabarium micranthum* , *Asparagus cochinchinensis* , *Lonicera macrantha* , *Semiaquilegia adoxoides* , *Gastrodia elata* , *Asparagus cochinchinensis, Asparagus lucidus, Magnolia sieboldii, Aesculus wilsonii, Prunus amygdalus dulcis protein, Beta vulgaris* , *Rubus suavissimus* , *Citrus aurantium dulcis* , *Citrus sinensis, Cucumis melo , Stevia rebaudiana* , *Eupatorium rebaudianum* , *Porphyra yezoensis, Gentiana manshurica, Murraya koenigii* , *Mesua ferrea* , *Dendrobium candidum* , *Adiantum capillus veneris* , *Pheretima vulgaris , Geranium thunbergii, Lespedeza capitata, Pelargonium capitatum, geranium, Phryma leptostachya, Speranskia tuberculata* , *Rosa damascena* , *Bolboma paniculatum* , *Eupolyphaga seu steleophaga, Evolvulus alsinoides, Tulipa kaufmanniana, Smilax glabra, Ipomoea hungaiensis, Pseudolarix amabilis , Hypericum patulum, Inula helenium, Cuscuta chinensis, Pisum sativum, Polianthes tuberosa, Tagetes erecta, Vaccaria segetalis, Magnolia biondii, Pheretima guillelmi, Sargassum fulvellum, Micrococcus lysate, Sasa veitchii, Amaranthus caudatus* , *Potentilla chinensis* , *Curcuma wenyujin* , *Citrus unshiu, Cydonia oblonga* , *Pyrus cydonia* , *Xanthoceras sorbifolia*, *Equisetum arvense* , *Lactuca sativa , Diospyros ebenum* , *Vigna aconitifolia*, *Lindera strychnifolia*, *Cuttlefish*, *Epimedium wushanense , Sempervivum tectorum, Sapindus mukurossi, Pongamia glabra, Brassica rapa* , *Evodia rutaecarpa, Sterculia plantifolia*, *Galla rhois gallnut*, *Coleus scutellarioides* , *Acanthopanax gracilistylus, Meconopsis quintuplinervia, Schizandra chinensis* , *Arctium majus*, *Magnolia sprengeri* , *Myosotis sylvatica* , *Salvia hispanica* , *Tillandsia usneoides* , *Abies sibirica* , *Larix sibirica*, *Yucca schidigera , citrullus lanatus*, *Cucurbita pepo* , *Valeriana celtica*, *Rumex occidentalis*, *Panax quinquefolium, Sambucus nigra, Pyrus communis* , *Primula sikkimensis, Cinnamomum zeylanicum, Mourera fluviatilis , Siegesbeckia orientalis, Stachyurus himalaicus, Prunus serrulata, Mimosa tenuiflora, Euglena gracilis polysaccharide, Acorus gramineus* , *Mahonia fortunei* , *Leonurus sibiricus* , *Sphacelaria scoparia , Calanthe discolor, Shorea stenoptera butter, Bupleurum scorzonerifolium, Aframomum angustifolium , Cassia angustifolia*, *Artemisia dracunculus, Echinacea angustifolia* , *Vaccinium angustifolium , Prunella vulgaris* , *Tuber aestivum* , *Quercus robur, Satureia hortensis* , *Leucojum aestivum bulb , Agrimonia pilosa* , *Opuntia ficus-indica* , *Geranium robertianum* , *Ampelopsis grossedentata , Aspalathus linearis*, *Siegesbeckia pubescens* , *Nigella glandulifera*, *Bergenia ciliata*, *Ferula foetida , Mentha suaveolens*, *Isodon japonicus* , *Citrus junos* , *Amomum aromaticum* , *Melissa officinalis , Cyperus rotundus,, Iris florentina, Lentinus edodes mycelium, Vanilla planifolia, Zingiber aromaticus , viola odorata, Cymbopogon citratus, Prunus persica nectarina, Citrus aurantium bergamia, Mosla chinensis* , *Liatris odoratissima* , *Schisandra lancifloli, Dipteryx odorata, Rosa odorata* , *Nymphaea odorata* , *Myrtus communis*, *Lathyrus odoratus* , *Pelargonium graveolens*, *Pimenta acris* , *Lemon ekisu, Citrus medica limonum*, *Abies balsamea*, *Amyris balsamifera, Helianthus annuus* , *Evernia prunastri* , *Schinus terebinthifolius*, *Chrysanthemum parthenium* , *Freesia refracta*, *Elettaria cardamomum, Allium macroon, Broussonetia kazinoki, ouricury, Coffea arabica, Gleditsia australis, Combretum micranthum, Cirsium setosum, Cassia tora, Hypericum erectum, Spilanthes callimorpha , Triticum aestivum, Euphrasia officinalis, Capsicum frutescens, Clematis armandii, Arctium minus, chlorella vulgaris albus, Yucca glauca* , *Rumex acetosella* , *Tagetes minuta* , *Sargassum fusiforme*, *Camellia meiocarpa, Euphorbia cerifera, Laminaria ochroleuca, Valeriana officinalis, Allium chinensis, Tinospora cordifolia, Ajuga turkestanica, Arnebia euchroma, Astrocaryum vulgare, Gigartina stellata , Cimicifuga dahurica, Rubus chamaemorus, Armeniaca vulgaris, Prunus armeniaca* , *Arctostaphylos uva ursi* , *Fraxinus stylosa* , *Hydrangea macrophylla* , *Sparassis crispa* , *Spiraea salicifolia*, *Rosa rubiginosa* , *paulownia imperialis* , *Usnea barbota* , *Cynanchum paniculatum* , *Hemerocallis fulva, Scrophularia ningpoensis* , *Sargassum filipendula*, *Heterotheca inuloides* , *Inula japonica* , *Citrus aurantium sinensis, Galanthus nivalis* , *Saussurea involucrata* , *Cedrus deodara, Sambucus adnata , Lavandula angustifolia, Salvia lavandulaefolia*, *Caviar, Albatrellus confluens* , *Corthellus shiitake , Acacia farnesiana*, *Commelina communis* , *bacillus ferment, Camelina sativa* , *Linum usitatissimum , Cymbopogon nardus* , *Betula platyphylla japonica* , *Opuntia coccinellifera*, *Nicotiana tabacum , Tofieldia japonica* , *Empetrum nigrum, Vetiveria zizanoides, Cistus ladaniferus, Ophiopogon bodinieri , Lathyrus palustris, Rhus semialata gall, Agropyron repens, Pinus pumilio, Alpinia speciosa, Avena sativa*, *Passiflora henryi*, *Baptisia tinctoria* , *Hizikia fusiforme, Codonopsis lanceolata* , *Averrhoa carambola, Thymus zygis, Hedera helix*, *Allium cepa, Calophyllum tacamahaca, Robinia pseudacacia , Pseudanabaena galeata* , *Echium lycopsis*, *Rosa centifolia*, *Potentilla erecta* , *Zizyphus joazeiro , Anacardium occidentale*, *Althaea officinalis, arutea ekisu, Salvia officinalis* , *Fomes officinalis* , *Rheum officinale, Polygonatum officinale, Veronica officinalis, Parietaria officinalis, Fumaria officinalis, teacocoyl hydrolyzed collagen, Cocos nucifera, Fragaria vesca* , *Glycine soja, Scoparia dulcis, Pueraria lobota, Juglans cathayensis, Daucus carota, Chrysanthemum indicum* , *Melastoma candidum* , *Rhus succedanea, Rosa multiflora, Crataegus cuneata, Mallotus japonicus, Dipsacus sylvestris, Nyctanthes arbor-tristis, Triticum monococcum* , *Platanus occidentalis, Solidago decurrens, Cananga odorata, mussel, Myrciaria dubia, Urtica dioica* , *Leonurus artemisia, Alpinia oxyphylla, Cassia italica, Brassica oleracea italica*, *Helichrysum italicum*, *Pinus pinea, Coix lacryma-jobi ma-yuen* , *Moringa pterygosperma* , *Artemisia capillaris* , *Stellaria dichotoma lanceolata* , *Tremella fuciformis*, *Acacia dealbata, Tilia tomentosa, Chloranthus japonicus, Ginkgo biloba, Epimedium brevicornum, Bambusa arundinacea, Azadirachta indica, Melia azadirachta, Commiphora mukul resin, Garcinia indica butter, Schleichera trijuga, Swertia chirata, Plukenetia volubilis, Prunus speciosa, Cerasus pseudocerasus, Prunus pseudocerasus* , *Viburnum prunifolium* , *Spartium junceum* , *Actinidia chinensis hispida , Cordyceps militaris, Brassica campestris, Camellia oleifera, Olea europaea* , *Cyperus esculentus, Pinus tabulaeformis* , *Elaeis guineensis* , *Amorphophallus campanulatus* , *Smilax utilis* , *Citrus grandis* , *Phyllanthus emblica* , , *Houttuynia cordata* , *Spiraea ulmaria* , *Guazuma ulmifolia* , *Papaver rhoeas, Alchemilla vulgaris* , *Brassica oleracea acephala* , *Cycnoches cooperi* , *Haematococcus pluvialis* , *Iris ensata* , *Pimenta officinalis* , *Magnolia denudata bud, Zea mays starch, Polygonatum odoratum , Tulipa gesneriana, Prunus japonica, Guaiacum officinale, Coriandrum sativum* , *Juniperus chinensis xylem* , *Citrus japonica* , *Citrus madurensis* , *Mentha rotundifolia* , *Angelica archangelica* , *Drosera rotundifolia* , *Ipomoea purpurea* , *Triticum vulgare turgidum* , *Gypsophila paniculata* , *Polygala tenuifolia* , *Laurus nobilis* , *Rosa chinensis* , *Oenothera biennis* , *Vaccinium vitis-idaea* , *Styrax tonkinensis resin, Saussurea lappa, Coptis teeta, Paris polyphylla yunnanensis, Rosa hybrid, Trametes versicolor, Lavandula hybrida, Hordeum distichon* , *Lansium domesticum, Ziziphus jujuba* , *Gleditsia sinensis, Quillaja saponaria, Lycopus lucidus hirtus, Alisma orientale, Pistacia lentiscus* , *Premna fulva , swertia bimaculata* , *Cinnamomum camphora* , *Prunus pedunculata* , *Prunella asiatica* , *Ceratonia siliqua* , *Epimedium Grandiflorum* , *Dictyophora indusiata* , *Dioscorea villosa* , *Narcissus jonquilla , Corchorus olitorius* , *Poterium officinale* , *Kappaphycus alvarezii, Sanguisorba officinalis longifolia , Pinus palustris* , *Rheum palmatum* , *Palmaria palmata* , *Laminaria digitata* , *Rhodymenia palmata , Glycyrrhiza inflata* , *Canarium commune* , *Aesculus chinensis chekiangensis, Corylus heterophylla , Sesamum indicum, Eruca sativa, Anemarrhena asphodeloides, Gardenia florida, Gardenia jasminoides , Gardenia jasminoides, Ona sessilifolia, Fragaria chiloensis, Gevuina avellana, Rehmannia chinensis , Stachyurus chinensis, Ganoderma sinensis , Actinidia chinensis* , *Skeletonema costatum* , *Artemisia absinthium , Angelica pubescens, Chondrus crispus, Magnolia kobus, Rumex crispus, Calluna vulgaris , Halopteris scoparia , Cordyline terminalis , Hibiscus rosa-sinensis , Cyanotis arachnoidea , Polyporus umbellatus* , *Bamboo vinegar, Panax japonicus* , *Bamboo charcoal, Phyllostachys pubescens , Zanthoxylum alatum, Catalpa ovata, Lithospermum erythrorhizon, Syringa oblata, Petasites hybridus , Viola yedoensis* , *Chrysanthemum zawadskii* , *Peucedanum decursivum* , *Bletia hyacinthina bulb , Mirabilis jalapa* , *Medicago sativa , Porphyridium cruentum* , *Echinacea purpurea , Perilla frutescens , Pterocarpus santalinus , Wisteria sinensis , Lagerstroemia indica* , *Magnolia liliflora, Aster tataricus , Astragalus sinicus, Ganoderma sinensis, Alkanna tinctoria, Elaeis guineensis, Trachycarpus fortunei , Asparagus racemosus, et Cimicifuga racemosa.*

Dans le cas où le matériel biologique est une plante, l'extraction peut être effectuée à partir de l'ensemble de la plante ou d'une ou plusieurs parties de la plante, et notamment choisie parmi la racine, la tige, l'écorce, la fleur, la graine, le germe et/ou la feuille et leurs mélanges. Selon un mode avantageux, il s'agit préférentiellement des parties aériennes c'est-à-dire les feuilles et les tiges, et préférentiellement les feuilles.

Dans un mode de réalisation particulier, le matériel biologique est l'olivier, en particulier des feuilles d'olivier, l'orange, en particulier l'écorce de fruit d'oranger, la verveine officinale, en particulier ses tiges feuillées, ou le dartier (*Cassia alata*), en particulier ses feuilles.

### Composés extractibles

Une variété de composés ou substances peuvent être extraits en utilisant l'eau de coco comme solvant d'extraction. Les composés extractibles selon la présente invention incluent de manière non-exhaustive des terpènes, terpènoïdes, flavones and flavonoïdes, stéroïdes, stérols, saponines and sapogénines, alcanes, alcaloïdes, amines, acides aminés, aldéhydes, irrridoîdes, phénylpropanoïdes, alcools, polyols, lipides, acides gras, lignanes, phénols, pyrones, buténolides, lactones, chalcones, cétones, benzènes, cyclohexanes, glucosides, glycosides, cyanidines, furanes, phorbols, quinones et phloroglucinols sous formes aglycone ou le cas échéant sous forme glycosylée. L'invention peut également être appliquée à l'extraction de molécules bioactives de masse moléculaire élevée telles que des protéines, des peptides, des enzymes, des polysaccharides, des oligosaccharides et des glucides.

A titre d'exemple , les composés extractibles selon la présente invention incluent de manière non-exhaustive les substances suivantes : catéchine, épicatéchine, catéchine gallate, épicatéchine gallate, gallocatéchine, épigallocatéchine, gallocatéchine gallate, épigallocatéchine gallate, tanins cétéchiques, tanins galiques, rhamnétine, fisétine, robinétine, gossypétine, orientine, homoorientine, cirsiliol, acide homoprotocatechuique, acide dihydrocafféique, éthyl ester de l'acide protocatéchuique, propyl gallate, acide gallique, acide protocatechuique, acide cafféique, acide rosmarinique, esculétine, 4-methylesculétine, nordalbergine, acide chlorogénique, phénéthyl ester de l'acide cafféique, acide chicorique, échinacoside, beta-D-glucopyranoside, verbascoside, hydroxytyrosol, maclurine, 3,4-dihydroxybenzaldéhyde, 3,4-dihydroxybenzophénone, butéine, 3,4-dihydroxyacétophénone, maréine, ériodictyolchalcone, pyrocatéchol, acide nordihydroguaiaretique, 3-hydroxydaidzéine, oleuropéine, maritiméine, acide salicylique, myristicine, eugenol, ombelliférone, aesculetine, juglone, resvératrol, kaempférol, afzéline, astragaline, juglanine, robinine, trifoline, kaempférol-3-O-rutinoside, daidzine, puérarine, procyanidines, dalphiniol, herniarine, skimmine; marmarine, marmésine, angélicine, impératorine, xanthotoxine, bergaptène, psoralène, linalol, 1,8-cinéole, alpha-pinène, bêta-sitostérol, campestrol, stigmastérol, acides de fruits (acide lactique, acide glycolique, acide malique, acide citrique...), vitamine E (tocophérols), vitamine A (rétinoïdes), provitamine A (caroténoïdes), vitamine C, vitamines B (thiamine, biotine, nicotinamide, acide panthothénique...), camphre, menthol, myrcène, abyssinone I, abyssinone V, afzelechine, ampelopsine, aromadendrine, auriculoside, broussine, broussonin C, butine, butrine, davidigenine, diffutine, 7,4'- dihydroxylflavane, 2,6-dihydroxyl-4'-methoxydihydro-chalcone, 7,3'-dihydroxyl-4-methoxy-8-methylflavane, 7,4'-dihydroxyl-8-methylfalvane, 6,8-diprenylnaringenine, dracorubine, eriocitrine, eriodictyol, farrerol, fisetinidol, fisetinidol-4-ol, fustine, garbanzol, glabranine, glepidotine β, glycyphylline, hesperetine, hesperidine, homoeriodictyol, 7-hydroxyflavane, isochamaejasmine, isosakuranetine, isouriaretine, kolaflavanone, liquiretigenine, manniflavanone,-methoxytaxifoline, narirutine, neoastilbine, neoeriocitrine, neohesperidine, phloretine, phellamurine, phloridzine, pinobanksine, pinocembrine, pinocembrine 7-rhamnosyl-glucoside, piperaduncine β, poncirine, prenylnaringenine, sakuranetine, silandrine, silybine, silychristine, sophoranone, strobopinine, taxifolene, taxifoline-3-O-acetate, tephrowatsine, theasinensine A, uvaretine, naringenine, naringenine glycosides, oglucosylrutine, alpha - glucosylmyricetine, alpha-glucosylisoquercetine, alpha-glucosylquercetine, dihydroquercetine (taxifoline), hesperidine (3',5,7-trihydroxy-4'-methoxyflavanone 7-rhamnoglucoside, hesperitine 7-0-rhamnoglucoside), neohesperidine, rutin (3,3',4',5,7-pentahydroxyflavone 3-rhamnoglucoside, quercetin 3-rhamnoglucoside), diosmine (3',4',7-trihydroxy-5-methoxyflavanone 7-rhamnoglucoside), eriodictine, apigenine 7-glucoside (4',5,7-trihydroxyflavone 7-glucoside), kaempferol, quercitrine, avicularine, myricitine.

### Extraits et Utilisations

Un extrait est obtenu par le procédé et utilisé. L'extrait se caractérise par la présence d'eau de coco. Ainsi, l'extrait est obtenu par la méthode d'extraction selon la présente invention sans étape d'élimination de l'eau de coco notamment sans étape de purification ni fractionnement.

L'extrait est utilisé pour la fabrication d'une composition nutraceutique, d'un produit diététique ou alimentaire, d'un complément nutritionnel, d'une composition pharmaceutique notamment dermatologique ou d'une composition cosmétique. La presente description est également relative à une composition nutraceutique, d'un produit diététique ou alimentaire, d'un complément nutritionnel, d'une composition pharmaceutique notamment dermatologique ou d'une composition cosmétique comprenant un extrait obtenu par le procédé selon la présente invention.

La présente invention est également relative à une méthode de préparation d'une composition nutraceutique, diététique, alimentaire, nutritionnelle, pharmaceutique notamment dermatologique ou cosmétique comprenant la préparation d'un extrait de matériel biologique végétal, animal et/ou procaryote selon la méthode selon la présente invention et l'incorporation de l'extrait ainso obtenu dans une composition nutraceutique, diététique, alimentaire, nutritionnelle, pharmaceutique notamment dermatologique ou cosmétique. De manière avantageuse, la composition ainsi obtenue contient de l'eau de coco ayant servi de solvant d'extraction de l'extrait.

L'extrait est ainsi oralement et topiquement acceptable. L'extrait obtenu est préférentiellement présent dans la composition cosmétique, nutraceutique et/ou pharmaceutique, en particulier dermatologique à une concentration de 1.10⁻⁴% à 10% en poids, préférentiellement entre 1.10⁻⁴% et 5% en poids, encore avantageusement entre 1.10⁻³% et 3% en poids par rapport au poids total de la composition, en particulier entre 0,001 et 0,1 % en poids par rapport au poids total de la composition.

Les compositions cosmétiques, nutraceutiques ou pharmaceutiques, préférentiellement dermatologiques peuvent contenir tout solvant approprié et/ou tout véhicule cosmétique, nutraceutique ou pharmaceutique, préférentiellement dermatologique approprié et/ou tout excipient approprié, éventuellement en combinaison avec d'autres composés d'intérêts. Les termes "véhicule cosmétique, nutraceutique, pharmaceutique ou dermatologique approprié", utilisés ici, signifient que la composition ou les composants de celle-ci sont adaptés à l'utilisation en contact avec la peau humaine ou par voie orale sans toxicité, incompatibilité, instabilité, réponse allergique, ou leurs équivalents, indue.

Avantageusement, les compositions sont formulées sous une forme choisie parmi le groupe consistant en une solution, aqueuse ou huileuse, une crème ou un gel aqueux ou un gel huileux, notamment en pot ou en tube, notamment un gel douche, un shampoing ; un lait ; une émulsion, une microémulsion ou une nanoémulsion, notamment huile-dans-eau ou eau-dans-huile ou multiple ou siliconée ; une lotion, notamment en flacon de verre, de plastique ou en flacon doseur ou en aérosol ; une ampoule ; un savon liquide ; un pain dermatologique ; une pommade ; une mousse ; un produit anhydre, de préférence liquide, pâteux ou solide, par exemple sous forme de bâtonnet; des poudres.

Les compositions cosmétiques peuvent être appliquées par voie topique, sur une surface du corps choisie parmi la peau du visage et/ou du corps ou la propriété cosmétique de l'extrait obtenu est souhaitable, par exemple sur une zone du corps inconfortable, inesthétique et/ou désagréable, tel que le front, les joues et /ou le menton, les zones du corps tels que le cuir chevelu, les bras, les mains, les jambes, le ventre et/ou le buste.

Pour les compositions, l'excipient contient par exemple au moins un composé choisi parmi le groupe consistant en les conservateurs, les émollients, les émulsifiants, les tensioactifs, les hydratants, les épaississants, les conditionneurs, les agents matifiants, les stabilisants, les antioxydants, les agents de texture, les agents de brillance, les agents filmogènes, les solubilisants, les pigments, les colorants, les parfums et les filtres solaires. Ces excipients sont de préférence choisis parmi le groupe consistant en les acides aminés et leurs dérivés, les polyglycérols, les esters, les polymères et dérivés de cellulose, les dérivés de Lanoline, les phospholipides, les lactoferrines, les lactoperoxidases, les stabilisants à base de sucrose, les vitamines E et ses dérivés, les cires naturelles et synthétiques, les huiles végétales, les triglycérides, les insaponifiables, les phytosterols, les esters végétaux, les silicones et ses dérivés, les hydrolysats de protéines, l'huile de Jojoba et ses dérivés, les esters lipo/hydrosolubles, les betaines, les aminoxides, les extraits de plantes les esters de Saccharose, les dioxydes de Titane, les glycines, et les parabens, et encore de préférence parmi le groupe consistant en le butylène glycol, le stéareth-2, le stéareth-21, le glycol-15 stéaryl éther, le cétéaryl alcool, le phénoxyéthanol, le méthylparaben, l'éthylparaben, le propylparaben, le butylparaben, le butylène glycol, les tocopherols naturels, la glycérine, le dihydroxycetyl sodium phosphate, l'isopropyl hydroxycétyl éther, le glycol stéarate, le triisononanoin, l'octyl cocoate, le polyacrylamide, l'isoparaffine, le laureth-7, un carbomer, le propylène glycol, le glycérol, le bisabolol, une diméthicone, l'hydroxyde de sodium, le PEG 30-dipolyhydroxystérate, les caprique/caprylique triglycérides, le cétéaryl octanoate, le dibutyl adipate, l'huile de pépin de raisin, l'huile de jojoba, le sulfate de magnésium, l'EDTA, une cyclométhicone, la gomme de xanthane, l'acide citrique, le lauryl sulfate de sodium, les cires et les huiles minérales, l'isostéaryl isostéarate, le dipélargonate de propylène glycol, l'isostéarate de propylène glycol, le PEG 8, Beeswax, les glycérides d'huile de coeur de palme hydrogénée, les glycérides d'huile de palme hydrogénée, l'huile de lanoline, l'huile de sésame, le cétyl lactate, le lanoline alcool, l'huile de ricin, le dioxyde de titane, le lactose, le saccharose, le polyéthylène basse densité, une solution isotonique salée.

De nombreux ingrédients cosmétiquement actifs connus par l'homme du métier pour améliorer la santé et/ou l'apparence physique de la peau peuvent être contenus dans les compositions cosmétiques. L'homme du métier sait formuler les compositions cosmétiques ou dermatologiques pour obtenir les meilleurs effets. D'autre part les composés décrits dans la présente invention peuvent avoir un effet de synergie lorsqu'ils sont combinés les uns aux autres. Ces combinaisons sont également couvertes par la présente invention. Le CTFA Cosmetic Ingrédient Handbook, Second Edition (1992) décrit différents ingrédients cosmétiques et pharmaceutiques utilisés couramment dans l'industrie cosmétique et pharmaceutique, qui sont en particulier adaptés à une utilisation topique. Des exemples de ces classes d'ingrédients comprennent, sans en être limité les composés suivants: abrasif, absorbants, composé à but esthétique tel que les parfums, les pigments, les colorants, les huiles essentielles, les astringents, etc. (par exemple: l'huile de clou de girofle, menthol, camphre, l'huile d'eucalyptus, eugénol, menthyl lactate, distillat d'hamélis), les agents anti-acné, les agents anti-floculants, les agents antimousse, les agents antimicrobiens (par exemple: iodopropyl butylcarbamate), les antioxydants, les liants, les additives biologiques, les agents tampon, les agents gonflants, les agents chélatants, les additifs, les agents biocides, les dénaturants, les épaississants, et les vitamines, et les dérivés ou équivalents de ceux-ci, les matériaux formant des films, les polymères, les agents opacifiants, les ajusteurs de pH, les agents réducteurs, les agents dépigmentants ou éclaircissants (par exemple : hydroquinone, acide kojique, acide ascorbique, magnésium ascorbyl phosphate, ascorbyl glucosamine), les agents de conditionnement (par exemple : les humectants). D'autres buts, caractéristiques et avantages de l'invention apparaîtront clairement à l'homme de l'art suite à la lecture de la description explicative qui fait référence à des exemples qui sont donnés seulement à titre d'illustration et qui ne sauraient en aucune façon limiter la portée de l'invention. Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité. Ainsi, chaque exemple a une portée générale.

D'autre part, dans les exemples, tous les pourcentages sont donnés en poids, sauf indication contraire, la température est exprimée en degré Celsius sauf indication contraire, et la pression est la pression atmosphérique, sauf indication contraire.

### EXEMPLES

### EXEMPLE 1 - Effet de différentes eaux de constitution sur l'extraction de polyphénols totaux des feuilles d'olivier et des écorces de fruit d'oranger.

### Exemple 1-1- Eaux végétales

Plusieurs eaux végétales naturelles ont été testées.

### Eaux de constitution de carotte et de salade

Les eaux de carotte et de salade ont été extraites en utilisant la technique d'Hydrodiffusion par micro-ondes et gravité (Microwave Hydrodiffusion and Gravity ou MHG). Cette technique qui combine à la fois le chauffage par micro-ondes et la gravité présente l'avantage de pouvoir être transposable à l'échelle industrielle. Elle est notamment décrite dans le brevet EP1955749 B1. Cette extraction est réalisée sans solvant. L'action des micro-ondes fait chauffer la matrice jusqu'à éclatement des cellules. Ainsi, le contenu cellulaire, dont les métabolites, sont libérés et entrainés avec l'eau intrinsèque par hydrodiffusion. La vapeur formée se condense et la gravité va permettre à l'extrait de descendre dans le réfrigérant. L'eau de constitution est alors recueillie.

Les conditions d'extraction des eaux de carotte et de l'eau de salade par MHG ont été optimisées et établies à 20 minutes avec une puissance de 1W/g (avec un maximum de 300W).

Les deux autres fluides végétaux testés (eau de coco et jus *d'Aloe vera*) ont été achetés dans le commerce. Trois eaux de coco d'origine différente ont été testées : eau de coco A, eau de coco B et eau de coco C selon les exemples 1, 2, 3 et 4.

### Exemple 1-2- Extraction des polyphénols de feuilles d'olivier

Les feuilles d'olivier (*Olea europaea* L.) séchées et broyées ont été extraites pendant 1 heure à 60°C (ratio plante/solvant = 1/10) dans l'eau et dans chacune des eaux de constitution étudiées.

Après refroidissement, centrifugation et filtration à 0,45 µm sur filtres PTFE, les polyphénols totaux ont été dosés dans les extraits par la méthode de Folin Ciocalteu par spectromètrie UV-Vis en utilisant l'oleuropéine comme molécule étalon.

Les résultats exprimés en mg d'oleuropéine/g de Matières Sèches sont présentés dans le tableau I.

Les extractions ont été réalisées en triplicatas et les résultats représentent la moyenne des valeurs déterminées.

**Tableau I : Comparaison de l'eau distillée et des eaux de constitution comme solvant d'extraction des polyphénols totaux de feuilles d'olivier.**

| Solvant d'extraction | Polyphénols totaux (mg d'oleuropéine/g de Matière Sèche) | Variation par rapport à l'extrait aqueux |
|---|---|---|
| Eau distillée | 14,6 | - |
| Eau de salade | 12,7 | -13 % |
| Eau de carotte | 11,5 | -21 % |
| Eaux de coco A | 29,4 | +101 % |
| Eau d'*Aloe vera* | 12,5 | -14 % |

Ces résultats montrent que, de manière inattendue et surprenante, l'utilisation de l'eau de coco comme solvant d'extraction permet d'extraire d'avantage de polyphénols totaux à partir des feuilles d'olivier par rapport à l'eau seule.

### Exemple 1-3- Extraction des polyphénols d'écorce d'orange

Les écorces de fruits d'oranger (*Citrus aurantium* L.) séchées et broyées ont été extraites et traitées dans les mêmes conditions que les feuilles d'olivier de l'exemple 1.

Les polyphénols totaux ont été dosés dans les extraits par la méthode de Folin Ciocalteu par spectromètrie UV-Vis en utilisant la naringine comme molécule étalon.

Les résultats exprimés en mg de naringine/g de Matières Sèches sont présentés dans le tableau II.

Les extractions ont été réalisées en triplicatas et les résultats représentent la moyenne des valeurs déterminées.

**Tableau II : Comparaison de l'eau distillée et des eaux de constitution comme solvant d'extraction des polyphénols totaux d'écorce de fruit d'oranger.**

| Solvants | Polyphénols totaux (mg de naringine / g de mat. Sèche) | Variation par rapport à l'extrait aqueux |
|---|---|---|
| Eau distillée | 64,8 | - |
| Eau de salade | 74,0 | +14 % |
| Eau de carotte | 65,5 | + 1 % |
| Eau de coco A | 86,1 | + 33 % |

Ces résultats montrent que, comme dans le cas des feuilles d'olivier, l'eau de coco comme solvant d'extraction permet d'extraire d'avantage de polyphénols à partir des écorces de fruits d'oranger par rapport à l'eau seule.

Ces études montrent donc que, parmi les eaux végétales naturelles testées, l'eau de coco permet d'extraire d'avantage de polyphénols totaux par rapport à l'eau seule.

### EXEMPLE 2 - Effet de l'eau de coco sur l'extraction de marqueurs phytochimiques à partir de différentes matrices végétales.

### Exemple 2-1- Extraction de l'oleuropéine à partir de feuilles d'olivier

Les feuilles d'olivier séchées et broyées ont été ajoutées (ratio plante/solvant = 1/10) sous agitation au solvant d'extraction (eau, eaux de coco) dont le pH a été ajusté à 3 ou à 4, pendant 1 heure à 80°C. Le récipient servant à l'extraction a été fermé (bouchon ou papier aluminium) pour éviter l'évaporation du solvant.

Après refroidissement, centrifugation et filtration à 0,45 µm, la concentration en oleuropéine dans les extraits liquides a été estimée directement par analyse en HPLC.

Les résultats exprimés en mg d'oleuropéine/litre d'extrait sont présentés dans le tableau III.

Les extractions ont été réalisées en triplicatas et les résultats représentent la moyenne des valeurs déterminées.

**Tableau III : Comparaison de l'eau distillée et de l'eau de coco comme solvant d'extraction de l'oleuropéine**

| | pH3 | | pH4 | |
|---|---|---|---|---|
| Solvants | [oleuropéine] en mg/L | Variation par rapport à l'extrait aqueux | [oleuropéine] en mg/L | Variation par rapport à l'extrait aqueux |
| Eau distillée | 234,5 | - | 203,0 | - |
| Eau de coco B | 322,5 | + 37,5 % | 275,5 | + 35,7 % |

### Exemple 2-2- Extraction de la naringine à partir d'écorces de fruit d'oranger

Les écorces de fruit d'oranger, séchées et broyées ont été ajoutées (ratio plante/solvant = 1/10) sous agitation au solvant d'extraction (eau, eau de coco) dont le pH a été ajusté à 3 ou à 4, pendant 1 heure à 80°C en milieu fermé pour éviter l'évaporation du solvant.

Après refroidissement, centrifugation et filtration à 0,45 µm, la concentration en naringine dans les extraits liquides a été estimée directement par analyse en HPLC.

Les résultats exprimés en mg de naringine/litre d'extrait sont présentés dans le tableau IV.

Les extractions ont été réalisées en triplicatas et les résultats représentent la moyenne des valeurs déterminées.

**Tableau IV : Comparaison de l'eau distillée et de l'eau de coco comme solvant d'extraction de la naringine**

| | pH3 | | pH4 | |
|---|---|---|---|---|
| Solvants | [naringine] en mg/L | Variation par rapport à l'extrait aqueux | [naringine] en mg/L | Variation par rapport à l'extrait aqueux |
| Eau distillée | 50,5 | - | 58,0 | |
| Eau coco B | 106,5 | + 110,9 % | 94,5 | + 62,9 % |

### EXEMPLE 3 - Extraction du verbascoside à partir de tiges feuillées de verveine officinale

Les tiges feuillées de verveine officinale (*Verbena officinalis* L) séchées et broyées ont été ajoutées (ratio plante/solvant = 1/10) sous agitation au solvant d'extraction (eau, eau de coco) dont le pH est ajusté à 3 ou à 4, pendant 1 heure à 80°C en milieu fermé pour éviter l'évaporation du solvant.

Après refroidissement, centrifugation et filtration à 0,45 µm, la concentration en verbascoside dans les extraits liquides a été estimée directement par analyse en HPLC.

Les résultats exprimés en mg de verbascoside/litre d'extrait sont présentés dans le tableau V.

Les extractions ont été réalisées en triplicatas et les résultats représentent la moyenne des valeurs déterminées.

**Tableau V : Comparaison de l'eau distillée et de l'eau de coco comme solvant d'extraction du verbascoside**

| | pH3 | | pH4 | |
|---|---|---|---|---|
| Solvants | [verbascoside] en mg/L | Variation par rapport à l'extrait aqueux | [verbascoside] en mg/L | Variation par rapport à l'extrait aqueux |
| Eau | 917 | | 696 | |
| Eau coco A | 1031 | + 12,4 % | 1063 | + 52,7 % |
| Eau coco B | 1077 | + 17,4 % | 1174 | + 68,7 % |

### EXEMPLE 4 - Extraction du kaempferol 3-O-sophoroside à partir de feuilles de Cassia alata

Les feuilles de *Cassia alata* L. séchées et broyées ont été ajoutées (ratio plante/solvant = 1/10) sous agitation au solvant d'extraction (eau, eau de coco) dont le pH est ajusté à 3 ou à 4, pendant 1 heure à 80°C en milieu fermé pour éviter l'évaporation du solvant.

Après refroidissement, centrifugation et filtration, à 0,45 µm, la concentration en kaempferol 3-O-sophoroside (K3OS) dans les extraits liquides a été estimée directement par analyse en HPLC.

Les résultats exprimés en mg de K3OS/litre d'extrait sont présentés dans le tableau VI.

Les extractions ont été réalisées en triplicatas et les résultats représentent la moyenne des valeurs déterminées.

**Tableau VI : Comparaison de l'eau distillée et de l'eau de coco comme solvant d'extraction du Kaempferol 3-O-sophoroside**

| | pH3 | | pH4 | |
|---|---|---|---|---|
| Solvants | [K3OS] en mg/L | Variation par rapport à l'extrait aqueux (%) | [K3OS] en mg/L | Variation par rapport à l'extrait aqueux (%) |
| EtOH/Eau 80:20 | 1890 | | | |
| Eau | 1180 | | 1140 | |
| Eau coco A | 1360 | 15 | 1320 | 16 |
| Eau coco B | 1440 | 22 | 1420 | 25 |

Les résultats obtenus montrent que l'eau de coco, quelle que soit son origine et le pH étudié, permet d'extraire les composés ciblés en concentration supérieure par rapport à l'eau distillée seule.

### EXEMPLE 5 - Extraction par l'eau de coco en conditions subcritiques

Ces essais ont été réalisés avec une eau de coco commerciale C.

10 grammes d'écorces de fruits d'oranger broyés ont été introduits dans un filtre à thé qui a été placé dans un réacteur avec 100 ml de solvant (eau ou eau de coco). L'extraction a été réalisée par l'intermédiaire d'un réacteur microondes UltraClave (Milestone). L'oxygène a été éliminé par introduction d'azote qui sert à la mise sous pression du réacteur. Les extractions ont été réalisées à une pression de 30 bars, pendant 10 minutes et à différentes températures. A la fin des extractions, le milieu a été filtré puis analysé.

Les polyphénols totaux sont dosés dans les extraits par la méthode de Folin Ciocalteu par spectrométrie UV-Vis en utilisant la naringine comme molécule étalon. Les résultats exprimés en mg de naringine/g de Matières Sèches sont présentés dans le tableau VII.

**Tableau VII: Comparaison de l'eau distillée et de l'eau de coco en conditions subcritiques comme solvants d'extraction de la naringine d'écorces de fruit d'oranger.**

| | Polyphénols totaux (mg de naringine / g de mat. Sèche) | | |
|---|---|---|---|
| Température | Eau | Eau de coco C | Variation par rapport à l'eau (%) |
| 105 °C | 31,0 | 37,1 | + 19,7 % |
| 115°C | 21,2 | 40,2 | + 89,6 % |
| 125°C | 23,8 | 51,2 | + 115,1 % |

Les résultats obtenus montrent que l'eau de coco permet d'extraire les polyphénols en concentration supérieure par rapport à l'eau distillée seule. En outre, avec l'eau distillée, on observe un brunissement des extraits qui n'est pas observé avec l'eau de coco.

## Revendications

1. Utilisation de l'eau de coco comme solvant d'extraction, en particulier pour l'extraction de composés ou de préparation d'extraits à partir de matériel biologique végétal, animal et/ou procaryote.

2. Méthode d'extraction de composés ou de préparation d'extraits à partir de matériel biologique végétal, animal et/ou procaryote, comprenant l'incubation dudit matériel biologique végétal, animal et/ou procaryote avec de l'eau de coco, et la récupération des composés ou de l'extrait.

3. Méthode selon la revendication 2, **caractérisée en ce que** la méthode comprend une étape d'immersion du matériel biologique dans l'eau de coco; une étape d'extraction du mélange obtenu ; et une étape de récupération des composés ou de l'extrait comprenant une étape de centrifugation, de filtration, d'évaporation, de concentration, de fractionnement ou de purification ou une combinaison de ces étapes.

4. Méthode selon la revendication 2 ou 3, **caractérisée en ce que** l'eau de coco et le matériel biologique sont mis en oeuvre avec un ratio pondéral compris entre 1: 1 et 100 : 1, de préférence entre 2 : 1 et 100 : 1 ou entre 5 : 1 et 20 : 1, par exemple à un ratio de 10 : 1.

5. Méthode selon la revendication 2 ou 3, **caractérisée en ce qu'**un tensioactif anionique, cationique ou non ionique est ajouté à l'eau de coco pour faciliter l'extraction des composés.

6. Méthode selon l'une quelconque des revendications 2 à 5, **caractérisée en ce que** l'étape d'extraction est réalisée à une température comprise entre 2°C et 100 °C, de préférence entre 20°C et 90°C, par exemple entre 50°C et 80°C pendant 15 minutes à 2 jours, de préférence pendant 30 minutes à 1 jour, et en particulier pendant 1 à 3 heures.

7. Méthode selon l'une quelconque des revendications 2 à 5, **caractérisée en ce que** l'étape d'extraction est réalisée en conditions subcritiques.

8. Méthode selon la revendication 7, **caractérisée en ce que** l'extraction est réalisée sous azote et sous pression à des températures comprises entre 100 et 140 °C pendant 5 minutes à 1 heure.

9. Méthode selon l'une quelconque des revendications 2-8, **caractérisée en ce que** l'étape d'extraction est assistée par microondes ou par utrasons.

10. Méthode selon l'une quelconque des revendications 2-9, **caractérisée en ce que** le matériel biologique est une plante ou une ou des parties de celle-ci.

11. Méthode selon l'une quelconque des revendications 2-10, **caractérisée en ce que** les composés extraits incluent des terpènes, terpènoïdes, flavones and flavonoïdes, stéroïdes, stérols, saponines and sapogénines, alcanes, alcaloïdes, amines, acides aminés, aldéhydes, irrridoîdes, phénylpropanoïdes, alcools, polyols, lipides, acides gras, lignanes, phénols, pyrones, buténolides, lactones, chalcones, cétones, benzènes, cyclohexanes, glucosides, glycosides, cyanidines, furanes, phorbols, quinones et phloroglucinols sous formes aglycone ou le cas échéant sous forme glycosylée et des molécules bioactives de masse moléculaire élevée telles que des protéines, des peptides, des enzymes, des polysaccharides, des oligosaccharides et des glucides.

12. Méthode de préparation d'une composition nutraceutique, diététique, alimentaire, nutritionnelle, pharmaceutique notamment dermatologique ou cosmétique comprenant la préparation d'un extrait de matériel biologique végétal, animal et/ou procaryote selon la méthode selon l'une quelconque des revendications 2-11 et l'incorporation de l'extrait dans une composition nutraceutique, diététique, alimentaire, nutritionnelle, pharmaceutique notamment dermatologique ou cosmétique.

## Patentansprüche

1. Verwendung von Kokosnusswasser als Extraktionslösungsmittel, insbesondere zur Extraktion von Verbindungen oder zur Herstellung von Extrakten aus pflanzlichem, tierischem und/oder prokaryotischem biologischem Material.

2. Verfahren zur Extraktion von Verbindungen oder zur Herstellung von Extrakten aus pflanzlichem, tierischem und/oder prokaryotischem biologischem Material, umfassend die Inkubation des pflanzlichen, tierischen und/oder prokaryotischen biologischen Materials mit Kokosnusswasser und die Wiedergewinnung der Verbindungen oder des Extrakts.

3. Verfahren gemäß Anspruch 2, **dadurch gekennzeichnet, dass** das Verfahren einen Schritt des Eintauchens des biologischen Materials in Kokosnusswasser; einen Schritt der Extraktion des erhaltenen Gemisches; und einen Schritt der Wiedergewinnung der Verbindungen oder des Extrakts umfasst, umfassend einen Zentrifugations-, Filtrations-, Verdampfungs-, Konzentrierungs-, Fraktionierungs- oder Reinigungsschritt oder eine Kombination dieser Schritte.

4. Verfahren gemäß Anspruch 2 oder 3, **dadurch gekennzeichnet, dass** das Kokosnusswasser und das biologische Material in einem Gewichtsverhältnis zwischen 1:1 und 100:1, vorzugsweise zwischen 2:1 und 100:1 oder zwischen 5:1 und 20:1, zum Beispiel in einem Verhältnis von 10:1 verwendet wird.

5. Verfahren gemäß Anspruch 2 oder 3, **dadurch gekennzeichnet, dass** ein anionisches, kationisches oder nichtionisches Tensid zu dem Kokosnusswasser gegeben wird, um die Extraktion der Verbindungen zu fördern.

6. Verfahren gemäß einem der Ansprüche 2 bis 5, **dadurch gekennzeichnet, dass** der Extraktionsschritt bei einer Temperatur zwischen 2°C und 100°C, vorzugsweise zwischen 20°C und 90°C, zum Beispiel zwischen 50°C und 80°C 15 Minuten bis 2 Tage, vorzugsweise 30 Minuten bis 1 Tag und insbesondere 1 bis 3 Stunden durchgeführt wird.

7. Verfahren gemäß einem der Ansprüche 2 bis 5, **dadurch gekennzeichnet, dass** der Extraktionsschritt unter subkritischen Bedingungen durchgeführt wird.

8. Verfahren gemäß Anspruch 7, **dadurch gekennzeichnet, dass** die Extraktion unter Stickstoff und unter Druck bei Temperaturen zwischen 100 und 140°C 5 Minuten bis 1 Stunde durchgeführt wird.

9. Verfahren gemäß einem der Ansprüche 2 bis 8, **dadurch gekennzeichnet, dass** der Extraktionsschritt durch Mikrowellen oder Ultraschall unterstützt wird.

10. Verfahren gemäß einem der Ansprüche 2 bis 9, **dadurch gekennzeichnet, dass** das biologische Material eine Pflanze oder ein oder mehrere Teile davon ist.

11. Verfahren gemäß einem der Ansprüche 2 bis 10, **dadurch gekennzeichnet, dass** die extrahierten Verbindungen Terpene, Terpenoide, Flavone und Flavonoide, Steroide, Sterole, Saponine und Sapogenine, Alkane, Alkaloide, Amine, Aminosäuren, Aldehyde, Iridoide, Phenylpropanoide, Alkohle, Polyole, Lipide, Fettsäuren, Lingnane, Phenole, Pyrone, Butenolide, Lactone, Chalcone, Ketone, Benzole, Cyclohexane, Glucoside, Glycoside, Cyanidine, Furane, Phorbole, Chinone und Phloroglucinole in Aglycon-Form oder gegebenenfalls in glykosylierter Form und hochmolekulare bioaktive Moleküle wie Proteine, Peptide, Enzyme, Polysaccharide, Oligosaccharide und Kohlenhydrate umfassen.

12. Verfahren zur Herstellung einer nutrazeutischen, diätetischen, alimentären, nutritionellen, pharmazeutischen, insbesondere dermatologischen oder kosmetischen Zusammensetzung, umfassend die Herstellung eines Extrakts aus pflanzlichem, tierischem und/oder prokaryotischem biologischem Material gemäß dem Verfahren gemäß einem der Ansprüche 2 bis 11 und Einbau des Extrakts in eine nutrazeutische, diätetische, alimentäre, nutritionelle, pharmazeutische, insbesondere dermatologische oder kosmetische Zusammensetzung.

## Claims

1. The use of coconut water as an extraction solvent, in particular for the extraction of compounds or for the preparation of extracts from plant, animal and/or prokaryotic biological material.

2. A method for the extraction of compounds or for the preparation of extracts from plant, animal and/or prokaryotic biological material, comprising the incubation of said plant, animal and/or prokaryotic biological material with coconut water, and the recovery of the compounds or of the extract.

3. The method according to claim 2, **characterized in that** the method comprises a step of immersing the biological material in coconut water; a step of extracting the mixture obtained; and a step of recovering the compounds or the extract comprising a step of centrifugation, filtration, evaporation, concentration, fractionation or purification or a combination of these steps.

4. The method according to claim 2 or 3, **characterized in that** the coconut water and the biological material are used in a weight ratio of between 1:1 and 100: 1, preferably between 2:1 and 100: 1 or between 5: 1 and 20: 1, for example in a ratio of 10:1.

5. The method according to claim 2 or 3, **characterized in that** an anionic, cationic or nonionic surfactant is added to the coconut water to facilitate the extraction of the compounds.

6. The method according to any one of claims 2 to 5, **characterized in that** the extraction step is performed at a temperature of between 2°C and 100°C, preferably between 20°C and 90°C, for example between 50°C and 80°C for 15 minutes to 2 days, preferably for 30 minutes to 1 day, and in particular for 1 to 3 hours.

7. The method according to any one of claims 2 to 5, **characterized in that** the extraction step is performed under subcritical conditions.

8. The method according to claim 7, **characterized in that** the extraction is performed under nitrogen and under pressure at temperatures of between 100 and 140°C for 5 minutes to 1 hour.

9. The method according to any one of claims 2-8, **characterized in that** the extraction step is assisted by microwaves or by ultrasound.

10. The method according to any one of claims 2-9, **characterized in that** the biological material is a plant or one or more parts thereof.

11. The method according to any one of claims 2-10, **characterized in that** the compounds extracted include terpenes, terpenoids, flavones and flavonoids, steroids, sterols, saponins and sapogenins, alkanes, alkaloids, amines, amino acids, aldehydes, iridoids, phenylpropanoids, alcohols, polyols, lipids, fatty acids, lignans, phenols, pyrones, butenolides, lactones, chalcones, ketones, benzenes, cyclohexanes, glucosides, glycosides, cyanidines, furans, phorbols, quinones and phloroglucinols in aglycone form or, where appropriate, in glycosyl form and bioactive molecules of high molecular mass such as proteins, peptides, enzymes, polysaccharides, oligosaccharides and carbohydrates.

12. A method for preparing a nutraceutical, dietetic, dietary, nutritional, pharmaceutical, notably dermatological, or cosmetic composition, comprising the preparation of an extract of plant, animal and/or prokaryotic biological material according to the method as claimed in any one of claims 2-11 and the incorporation of the extract into a nutraceutical, dietetic, dietary, nutritional, pharmaceutical, notably dermatological, or cosmetic composition.
